(19) Europäisches Patentamt / European Patent Office / Office européen des brevets

(11) **EP 4 117 706 B1**

(12) **EUROPEAN PATENT SPECIFICATION**

(45) Date of publication and mention
of the grant of the patent:
**04.09.2024 Bulletin 2024/36**

(21) Application number: **21710317.5**

(22) Date of filing: **11.03.2021**

(51) International Patent Classification (IPC):
*A61K 38/17* (2006.01)        *A61P 13/12* (2006.01)

(52) Cooperative Patent Classification (CPC):
**A61K 38/1741; A61P 13/12**

(86) International application number:
**PCT/EP2021/056253**

(87) International publication number:
**WO 2021/180884 (16.09.2021 Gazette 2021/37)**

(54) **FETUIN A FOR TREATMENT OF RENAL DISORDERS**

FETUIN A ZUR BEHANDLUNG VON NIERENERKRANKUNGEN

FÉTUINE-A POUR LE TRAITEMENT DE TROUBLES RÉNAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2020 EP 20162490**

(43) Date of publication of application:
**18.01.2023 Bulletin 2023/03**

(73) Proprietor: **Universität Bern**
**3012 Bern (CH)**

(72) Inventors:
• **HUYNH-DO, Uyen**
**3097 Liebefeld (CH)**
• **RUDLOFF, Stefan**
**3027 Bern (CH)**

(74) Representative: **CSL Global IP**
**C/o CSL Behring GmbH**
**Global Intellectual Property**
**Emil-von-Behring-Straße 76**
**35041 Marburg (DE)**

(56) References cited:
**WO-A1-2005/007199    WO-A2-02/094201**

• **DATABASE MEDLINE [online] US NATIONAL LIBRARY OF MEDICINE (NLM), BETHESDA, MD, US; December 2009 (2009-12-01), HÄUSLER MARTIN ET AL: "The physiologic development of fetuin-a serum concentrations in children.", Database accession no. NLM19690510**
• **HÄUSLER MARTIN ET AL: "The physiologic development of fetuin-a serum concentrations in children.", PEDIATRIC RESEARCH DEC 2009, vol. 66, no. 6, December 2009 (2009-12-01), pages 660 - 664, XP002799830, ISSN: 1530-0447, DOI: 10.1203/PDR.0b013e3181bc3f60**
• **RUDLOFF: "S wiss Medical Weekly Formerly: Schweizerische Medizinische Wochenschrift", SCHWEIZERISCHE MEDIZINISCHE WOCHENSCHRIFT, 19 November 2014 (2014-11-19), XP055717190**
• **WILLI JAHNEN-DECHENT ET AL: "Mineral chaperones: a role for fetuin-A and osteopontin in the inhibition and regression of pathologic calcification", JOURNAL OF MOLECULAR MEDICINE, SPRINGER, BERLIN, DE, vol. 86, no. 4, 15 December 2007 (2007-12-15), pages 379 - 389, XP019608762, ISSN: 1432-1440**
• **M KETTELER ET AL: "Association of low fetuin-A (AHSG) concentrations in serum with cardiovascular mortality in patients on dialysis: a cross-sectional study", THE LANCET, 1 January 2003 (2003-01-01), XP055717434**

EP 4 117 706 B1

- **HÄUSLER MARTIN ET AL: "The physiologic development of fetuin-a serum concentrations in children.", PEDIATRIC RESEARCH DEC 2009, vol. 66, no. 6, December 2009 (2009-12-01), pages 660 - 664, XP002799830, ISSN: 1530-0447**
- **RALF WESTENFELD ET AL: "Fetuin-A Protects against Atherosclerotic Calcification in CKD", JOURNAL OF THE AMERICAN SOCIETY OF NEPHROLOGY., vol. 20, no. 6, 1 June 2009 (2009-06-01), US, pages 1264 - 1274, XP055717189, ISSN: 1046-6673, DOI: 10.1681/ASN.2008060572**

Remarks:

The complete document including Reference Table(s) and the Sequence Listing(s) can be downloaded from the EPO website

**Description**

The present invention relates to Fetuin A (AHSG) for use in a method for treating a renal disorder, wherein an amount of Fetuin A effective for treating the renal disorder is administered to a subject in need thereof, wherein the renal disorder is an ischemic disorder. The present invention also relates to a pharmaceutical composition for use in treating a renal disorder, comprising Fetuin A and optionally at least one pharmaceutical acceptable carrier, wherein the renal disorder is an ischemic disorder.

An aim of modern medicine is to provide new treatments, including new targets in order to achieve better outcomes for particular patient groups. Those treatments, which take into account a patient's needs or risks, can rely on findings, targeting for example pathological signalling cascades in the cell or malfunction of cellular responses. Medicinal protein chemistry is a broad field, where physical, chemical, biological, bioinformatics and medical techniques are used to analyse and describe protein structures and mechanisms, identify fundamental molecular and genetic errors of disease and to develop interventions to correct them. This perspective emphasizes cellular and/or molecular phenomena and involvements rather than the former conceptual and observational focus on patients and their organs. Treatments with proteins require appropriate proteins and treatment recommendations, in order to identify those patients who benefit from certain therapeutic measures. Therefore, the development of treatments critically depends on the investigation of new molecular targeting drugs and procedures.

Ischemia reperfusion injury (IRI) is often involved in a wide range of pathologies, including ischemic stroke, myocardial infarction, acute kidney injury, cardiovascular surgery and organ transplantation. When the blood supply returns to the tissue after ischemia, this reoxygenation causes tissue damage. Paradoxically the as such necessary reoxygenation, exacerbates cellular dysfunction and apoptosis, following restoration of blood flow to formerly ischemic tissues. IRI occurs in a wide range of organs including the heart, lung, kidney, gut, skeletal muscle and brain and may involve not only the ischemic organ itself but may also induce systemic damage to distant organs, potentially leading to multi-system organ failure. Reperfusion injury is a multi-factorial process resulting in extensive tissue destruction. Initially, lack of sufficient blood flow results in deficiencies in oxygen supply, glucose and other substances required for normal mitochondrial metabolism, including reduced levels of ATP. This lack of ATP production causes cellular ATP-dependent ionic pumps (including the $Na^+/K^+$ and $Ca^{2+}$ pumps) to fail and the transmembrane ionic gradients get lost. Damaged or dying cells suffer calcium overload, which is characterized by calcium accumulation in mitochondria or apoptotic bodies, respectively. Concomitant low levels of ATP (malfunctioning mitochondria) and pyrophosphate levels (an important inhibitor of calcification) increases the calcification propensity of these organelles. A known complication in organ transplantation, which may also result from IRI, is delayed graft function (DGF). DGF describes a phenomenon where recipients that have undergone transplantation have a poor graft function during the first weeks after transplantation and need dialysis.

Therapeutic modalities to render organs more resistant to damages caused by ischemia and reperfusion have given controversial results or are just emerging. Therapeutic options to either enhance the resistance of organs against IRI or to rescue organs after sudden oxygen restriction, have been the subject of major research efforts in the past decades. Such strategies are, for example ischemic preconditioning, an experimental strategy in which exposure to short, non-lethal episodes of ischemia result in attenuated tissue injury during subsequent IRI, metabolic strategies to increase ischemia tolerance (glycolytic enzymes, erythropoeitin, etc. under the control of the transcription factor HIF-1$\alpha$), therapeutic gases, such as hydrogen, nitric oxide, hydrogen sulfide and carbon monoxide and also microRNAs (miRNAs) as therapeutic targets. Unfortunately, these modalities have given controversial results or are just emerging. Thus, treatment of renal disorders including, but not limited to IRI, remains an important unmet need.

Accordingly, there is a need for providing agents for treating renal disorders and the present invention offers a straightforward approach to address renal disorders, including ischemia reperfusion injury, in various settings.

Naturally occurring Fetuin A, also known as alpha-2-HS-glycoprotein (AHSG), is a liver-derived plasma glycoprotein, which is encoded by the *AHSG* gene. Fetuin A has been described in the art to attenuate early cerebral ischemic injury in a cerebral context, where IRI-driven inflammation has been diminished in rats with ischemic brain tissue (see, for example, Wang et. al, J Cereb Blood Flow Metab. 2010; 30(3): 493-504.). Fetuin A has further been related to the prevention of brain damage in stroke (WO 00/60943). However, it was also found that elevated plasma levels of Fetuin A increase the risk for myocardial infarction and ischemic stroke, showing that the role of Fetuin A as a prophylactic agent is not fully understood for ischemic brain damage (Weickert et. al, Circulation. 2008; 118: 2555-2562). Also, enhancing immunological response to cancer and cellular therapeutic methods for treating cancer and in particular to compounds, such as AHSG, and methods for downregulating the biological effects of TGF-$\beta$, has been discussed (WO 2005/007199).

Contradictory data has also been presented with regard to the use of Fetuin A as a biomarker for renal disorders. For example, recent studies, investigating the role of Fetuin A as biomarker in kidney transplantation, have failed to provide predictive information about the clinical outcome of patients (see, for example, Roos et. al, Kidney Blood Press Res. 2011; 34(5): 328-33; Mersai et. al, Urol J. 2015; 12(3): 2182-6; Koca et. al, Nephrology Dialysis Transplantation.

2017, 32(3): iii419-iii420). In said publications, Fetuin A levels generally were measured in the peripheral blood, which does not reflect Fetuin A levels in tissue. Taken together, these publications suggest that Fetuin A levels in blood are not a useful biomarker for long-term survival or clinical prognosis of patients, which suffer from renal disorders.

However, in the context of the present invention, it was surprisingly found that the administration of Fetuin A attenuates kidney tissue damage induced by hypoxic conditions in kidneys.

The invention is set out in the appended set of claims.

Therefore, in a first aspect, the present invention relates to a Fetuin A (AHSG) for use in a method for treating a renal disorder, wherein an amount of Fetuin A effective for treating the renal disorder is administered to a subject in need thereof, wherein the renal disorder is an ischemic renal disorder. Preferably, the renal disorder results from hypoxia, e.g. hypoxia during transplantation, cardiovascular surgery or other major surgery.

Without being bound by any theory and based on the experimental evidence provided herein, it is believed that when Fetuin A is administered to a subject suffering from an ischemic renal disorder, it acts as "mineral scavenger" in hypoxic renal tissues by "clearing" calcium-levels. Fetuin A is believed to counteract ectopic calcification to maintain renal tissue integrity and preventing inflammatory downstream cascades that may lead to renal fibrosis by IRI-induced expression of collagens. The herein proposed explanation is that Fetuin A, acts as a "mineral chaperone" in renal disorders, protects organs against ischemia reperfusion injury in kidney by clearing calcium-containing particles and mitigates the deleterious inflammatory response.

As used herein "renal disorder" is defined as one or more disorders or diseases affecting the kidney. The term encompasses renal diseases, but is not limited to them. For example, a renal injury that is not caused by a disease may also be a renal disorder (e.g. some injuries caused during major surgery; although major surgery may also be the cause a kidney diseases). The renal disorder is an ischemic renal disorder, i.e. a renal disorder resulting from and/or associated with ischemia. When a renal disorder "is" a certain disorder, this does not preclude the presence of other disorders and multiple other disorders may occur independently or interdependently at the same time as said renal disorder, e.g. IRI and DGF may occur simultaneously.

In a preferred embodiment of the invention, the renal disorder is selected from the group consisting of acute renal disorders, chronic renal disorders, kidney fibrosis, chronic kidney disease (CKD), renal insufficiency, renal inflammation, acute kidney injuries (AKI), disorders related to kidney hypoxia, renal ischemia-reperfusion injury (IRI), kidney tissue damage, disorders related to kidney transplantation, disorders related to cardiovascular surgery and combinations thereof, in particular wherein the kidney tissue damage is ischemic kidney tissue damage. A disorder related to cardiovascular surgery may for example be kidney tissue damage caused by cardiovascular surgery, in particular caused by hypoxia and/or ischemia during cardiovascular surgery.

In another embodiment of the invention, the ischemic renal disorder is selected from the group consisting of ischemic acute renal disorders, ischemic chronic renal disorders, kidney fibrosis resulting from ischemia, renal inflammation resulting from ischemia, acute kidney injuries resulting from ischemia, disorders related to kidney hypoxia, renal ischemia-reperfusion injury, ischemic kidney tissue damage, disorders related to ischemia during kidney transplantation (e.g. delayed graft function) and combinations thereof.

"Acute renal disorders" as used herein relate to a sudden functional loss or reduction of kidney function. This can be triggered by acute accidental kidney damage or acute occurring disorders. The causes for this are numerous, including, but not limited to pre-renal, post-renal and intrinsic acute kidney failure. Acute kidney failure can also be triggered by systemic diseases (such as a manifestation of an autoimmune disease), crush injury, contrast agents, some antibiotics and more.

"Chronic renal disorders" such as chronic kidney disease (CKD), chronic renal insufficiency or chronic renal inflammation, such as chronic pyelonephritis, are also included to a possible treatment spectrum, but not limited to those. "Chronic renal disorders" can either be triggered by anatomical location including, but not limited to those, vascular disease including large vessel disease such as bilateral kidney artery stenosis and small vessel disease such as ischemic nephropathy, hemolytic-uremic syndrome and vasculitis, glomerular disease, tubulointerstitial disease, obstructive nephropathy or congenital disease such as polycystic kidney disease or mesoamerican nephropathy.

"Fibrosis" relates to the formation of excess fibrous connective tissue, for example in a kidney or kidney tissue. Tissue inflammation may trigger fibrosis. In some embodiments, the fibrosis relates to interstitial fibrosis.

"Ischemia" or "ischemic" as used herein is an inadequate blood supply to an organ or specific part of the body. It is generally considered a severe clinical problem. Ischemia leads to a reduction in the supply of blood, hence results in a reduction of supply with oxygen and nutrients and also involves a reduced removal of waste products from tissues. This can result in irreversible tissue damage and cell death. Ischemia may occur in the context of and may be the cause of multiple renal disorders. Ischemia may also occur in the context of major surgery, e.g. it may be caused by major surgery. "Reperfusion" is herein defined as restoration of blood supply after an ischemic event. It may cause tissue damages by imbalancing, among others, the mineral balance, in particular by accumulation of calcium, e.g. in a kidney. "Ischemic reperfusion injury" (IRI), sometimes called reperfusion injury (RI) or re-oxygenation injury, is the tissue damage caused when blood supply returns to tissue (reperfusion) after a period of ischemia and/or after a period of reduced oxygen supply (hypoxia).

"Cardiovascular surgery" as used herein relates to the surgical treatment of great vessels and organs inside the thorax or abdomen, e.g. surgery involving heart, great vessels or lung. In some embodiments, cardiovascular surgery may be coronary artery bypass surgery or abdominal aortic aneurysm surgery. Abdominal aortic aneurysm surgery may cause renal ischemic reperfusion injury. Even if a cardiovascular surgery is not directly performed on the kidney, renal disorders may be associated with it. Ischemia during cardiovascular surgery may occur and cause kidney tissue damage. The development of acute kidney injury (AKI) after cardiovascular surgery is well known and some embodiments relate to the treatment of acute kidney injury resulting from cardiovascular surgery.

In context of the present invention, a "subject in need thereof" generally refers to either a human being, who is expected to suffer or is at risk to suffer from a renal disorder or a human being who already suffers from the renal disorder, in particular has already been diagnosed with an ischemic renal disorder. The subject may also be referred to as "patient". The subject may be in need of prophylactic or therapeutic treatment. The expectation to have an increased risk to suffer from a renal disorder may in some embodiments be due to a scheduled major surgery during which the occurrence of ischemia is a risk or even expected, e.g. an increased risk of a renal disorder in view of a scheduled kidney transplantation or cardiovascular surgery. In some embodiments, the subject in need thereof is expected to undergo or has undergone major surgery, preferably is expected to undergo or has undergone kidney transplantation or cardiovascular surgery.

"Treatment", "treat" or "treating" as used herein encompasses therapeutic treatment as well as prophylactic treatment (prevention) of a disorder. "Treatment", "treat" or "treating" may include administering a protein or compound described herein to thereby (i) reduce or eliminate at least one symptom of a specified disorder or (ii) to slow progression of the disorder or (iii) to stop or hinder the development of at least one symptom of a specified disorder. "Therapeutic treatment" and "therapeutic treating" as used herein refer to the treatment of an existing disorder, i.e. a treatment after the commencement of said disorder and its purpose is to eliminate, alleviate or slow down the existing disorder or to reduce the severity of it. In contrast, "prophylactic treatment", "prevention", "prevent" or "preventing" as used herein refer to a type of treatment for the disorder intended to reduce the risk, prevent or delay that a subject who does not presently have said disorder will develop it or symptoms and/or signs of it in the future, in particular after an event like a major surgery such as a transplantation. In a preferred embodiment, therapeutic treatment means improving the prognosis of the disorder. In some embodiments, prophylactic treatment is done before, during and/or after a major surgery such as a kidney transplantation or cardiovascular surgery to reduce the risk of onset for a disorder associated with major surgery, in particular associated with ischemia during kidney transplantation or cardiovascular surgery. In some further embodiments, the term "treating" or "treatment" preferably means that not only symptoms of the disorder are relieved but also that the disorder itself is improved, delayed or prevented, in particular that the disorder has a reduced severity or is prevented. Furthermore, "treatment" and "treating" may comprise the administration or the application of a therapeutic agent to a subject or patient or performance of a procedure or modality on a subject for obtaining a therapeutic or prophylactic benefit in relation to a disorder. For example, a treatment may include administration of a pharmaceutically effective amount of Fetuin A. In some embodiments, the above-mentioned disorder is a renal disease.

In an embodiment, the invention relates to Fetuin A for use in a method for treating a renal disorder, in particular therapeutically treating a renal disorder, wherein an amount of Fetuin A effective for treating the renal disorder is administered to a subject suffering from the renal disorder, wherein the renal disorder is an ischemic renal disorder. In this context, "treatment" or "treating" means the treatment of an established renal disorder and/or the treatment after the onset of said renal disorder.

In an alternative embodiment, the invention relates to Fetuin A for use in a method for prevention, also called prophylactic treatment, of a renal disorder, wherein an amount of Fetuin A effective for preventing the renal disorder is administered to a subject in need thereof, in particular to a subject who is at risk of suffering from the disorder in the future, e.g. a subject who is scheduled for major surgery that may involve ischemia of the kidney. In said alternative embodiment, "treatment" or "treating" mean "prevention" or "preventing" of a renal disorder before the onset of said disorder. In some cases at least one dose of Fetuin A is administered within 48 hours before a surgery, in particular transplantation surgery, comprising ischemia.

In another embodiment of the present invention, the renal disorder is a disorder related to, in particular caused by, kidney transplantation, in particular wherein said disorder is selected from the group consisting of delayed graft function (DGF) organ rejection of kidney transplants, kidney tissue damage resulting from kidney transplantation, inflammation resulting from kidney transplantation, ischemia reperfusion injury (IRI) resulting from kidney transplantation and combinations thereof. In some embodiments of the present invention, the renal disorder is a disorder caused by kidney transplantation, wherein said disorder is selected from the group consisting of delayed graft function, kidney tissue damage resulting from kidney transplantation, ischemia reperfusion injury resulting from kidney transplantation and combinations thereof.

In some embodiments, the renal disorder is an ischemic renal disorder resulting from kidney transplantation or cardiovascular surgery.

In another preferred embodiment of the present invention, the ischemic renal disorder is related to or caused by hypoxia, in particular wherein the hypoxia is caused by ischemia during major surgery, for example ischemia during cardiovascular surgery or kidney transplantation.

"Major surgery" as used herein is defined by an invasive operative procedure, in particular an invasive operative procedure in which a resection is performed. Major surgery may comprise that a body cavity is entered, organs are removed, organs are transplanted and/or normal anatomy is altered. In some embodiments, major surgery comprises local or general anaesthetization of the subject. In general, if a mesenchymal barrier is opened (pleural cavity, peritoneum, meninges), the surgery is considered "major". For example, kidney transplantation and cardiovascular surgery both are major surgeries. When the term "surgery" is used without specifying that it is a major surgery, this also comprises embodiments of "major surgery" in accordance with this invention.

In the context of the present invention, "kidney tissue damage" includes all forms of tissue damage, such as caused accidentally, caused by all cases of hypoxia, in particular hypoxia during kidney transplantation, cardiovascular surgery or other major surgery.

"Hypoxia" as used herein refers to a condition in which the body or a region of the body is deprived of adequate oxygen supply at the tissue level. It is often part of a pathological disorder. Hypoxia induced tissue damage includes all disorders, where oxygen supply is lost or diminished, causing oxygen undersupply in any tissue. This also includes reduced blood supply due to major surgery or blood vessel damages in general. The term "hypoxia" also includes anoxia as an extreme form of hypoxia, i.e. the total depletion in the level of oxygen.

In another preferred embodiment of the present invention, the IRI is caused by hypoxia during surgery, in particular major surgery. Hypoxic disorders, occurring in course of such a surgery, in particular a major surgery, can lead to ischemic reperfusion injury in the kidney, thereby damaging the tissue. The ischemic reperfusion injury at least partially may be caused by calcification.

In a further embodiment of the present invention, the renal disorder is delayed graft function, renal fibrosis or a combination thereof.

"Fetuin A" as used herein refers to a protein or fragment thereof or derivative thereof which either is naturally occurring Fetuin A or is not naturally occurring, but has the same or similar function as naturally occurring Fetuin A, in particular the same or similar function as naturally occurring human Fetuin A. Preferably, it is an inhibitor of calcification and/or the protein sequence is at least partially identical to human Fetuin A. In some embodiments, Fetuin A is a plasma derived or recombinant human Fetuin A. Human plasma derived Fetuin A is obtained from human plasma. The term Fetuin A shall also encompass all natural occurring alleles, splice variants and isoforms as well as synthetic peptides, peptide mimetics or peptide fragments, which have the same or similar functions as naturally occurring and/or human Fetuin A, such as inhibition of calcification.

In some embodiments of the present invention, the Fetuin A is human Fetuin A. In some embodiments, the human Fetuin A is derived from human blood plasma or, alternatively, the human Fetuin A is recombinant human Fetuin A. Plasma-derived human Fetuin A may be purified from human plasma. Recombinant human Fetuin A may have a different glycosylation pattern than plasma-derived human Fetuin A. In some embodiments Fetuin A is naturally occurring Fetuin A. A "naturally occurring protein" as referred to in the context of the present invention generally denotes a protein which is naturally found in any kind of living organism and which can as such be isolated from tissues, liquids, and/or from any sort of individual cells of said organism. For example, some naturally occurring proteins can be obtained from blood plasma of humans. Recombinantly produced proteins also include Fetuin A derivatives, which are somehow marked or labelled, for example by attaching gene-tags (such as FLAG-tag, Histag, Myc-tag, HA-tag or fluorescently labelled proteins such as GFP, YFP or RFP) that might or might not ease the purification.

"Purified" herein describes protein purification in general, which is well known to the skilled person in the art. In general, proteins can be purified based on their characteristics such as solubility, size, charge and specific binding affinity. That is, proteins are often separated based on their net charge by ion-exchange chromatography. Protein purification techniques are well known to the skilled person in the art. These techniques include, at one level, the homogenization and fractionation of the cells, tissue or organ to polypeptide and nonpolypeptide fractions. The protein or polypeptide of interest may be further purified using chromatographic and electrophoretic techniques to achieve partial or complete purification (or purification to homogeneity) unless otherwise specified. Methods particularly suited to the preparation of a pure peptide are ion-exchange chromatography, gel exclusion chromatography, polyacrylamide gel electrophoresis, affinity chromatography, hydrophobic interaction chromatography, immunoaffinity chromatography and isoelectric focusing. A method of purifying peptides is high-performance liquid chromatography. Another protein purification method is the fast protein liquid chromatography. For example, purification of plasma proteins can be performed by blood plasma fractionation and/or chromatographic steps. Protein purification may also include virus inactivation as a step. The term "purified" does not preclude the presence of some impurities, but in general, other blood components and/or impurities have been largely removed.

In another preferred embodiment of the invention the functionally active derivative or fragment of human Fetuin A is at least 70%, preferably at least 80%, more preferably at least 90%, even more preferably at least 95% or most preferably at least 99% identical and/or homologous to SEQ ID NO: 1, as defined below. "Homologue" is herein defined as sequence similarity within different biological sequences, on RNA, DNA and protein sequence levels. A "functionally active derivative", has the same or similar activity as naturally occurring Fetuin A, in particular may inhibit calcification. The percentage of sequence identity can be determined e.g. by sequence alignment. Methods of alignment of sequences for comparison are well known in the art. The NCBI Basic Local Alignment Search Tool (BLAST) (Altschul et al., 1990, J. Mol. Biol; 215:3, 403-410) is available from several sources, including the National Center for Biotechnology Information (NCBI, Bethesda, MD) and the Internet, for use in connection with the sequence analysis programs blastp, blastn, blastx, tblastn and tblastx.

Hence, in another embodiment of the present invention the Fetuin A comprises or consists of an amino acid sequence according to SEQ ID NO: 1 or a fragment or fragments thereof. In the context of the present invention, the term "a fragment thereof' or "fragments thereof' refer to any amino acid sequence or peptide defined by SEQ ID NO: 1, including, but not limited to, every smaller fragment revealing activity of Fetuin A or any fragment comprising additional amino acids, as defined above for the fragments and derivates of naturally occurring proteins with Fetuin A activity. In some embodiments the length of said fragment is at least 20%, at least 30%, at least 40%, at least 50% at least 60%, at least 70%, at least 80% or at least 90% of the length of SEQ ID NO: 1, wherein the length relates to the number of amino acids with respect to the full-length of SEQ ID NO: 1. Fragments may comprise additional amino acids that are not part of SEQ ID NO: 1. Fragments of the protein of the present invention can be obtained by sequence alterations in the protein (e.g. by one or more amino acid deletions, substitutions and/or additions), wherein the protein with the sequence alterations retains a function of the unaltered protein, namely its ability to localize during cell cycle progression to subcellular structures selected from the group consisting of the cell cortex, the contractile ring and the midbody or to function as a fluorescence reporter protein. Such sequence alterations can include, but are not limited to, conservative substitutions, deletions, mutations and/or insertions.

Human Fetuin A sequences (protein and nucleic acid sequences) are available from the "National Center for Biotechnology Information" (NCBI):

**SEQ ID** NO.: **1** (protein sequence of human Fetuin A)

```
MKSLVLLLCLAQLWGCHSAPHGPGLIYRQPNCDDPETEEAALVAIDYINQNLPWGYKHTLNQIDEVKV
WPQQPSGELFEIEIDTLETTCHVLDPTPVARCSVRQLKEHAVEGDCDFQLLKLDGKFSVVYAKCDSSP
ADSAEDVRKVCQDCPLLAPLNDTRVVHAAKAALAAFNAQNNGSNFQLEEISRAQLVPLPPSTYVEFTV
SGTDCVAKEATEAAKCNLLAEKQYGFCKATLSEKLGGAEVAVTCMVFQTQPVSSQPQPEGANEAVPTP
VVDPDAPPSPPLGAPGLPPAGSPPDSHVLLAAPPGHQLHRAHYDLRHTFMGVVSLGSPSGEVSHPRKT
RTVVQPSVGAAAGPVVPPCPGRIRHFKV
```

Protein Sequence: NP_001341500.1 (368 amino acids)
(https://www.ncbi.nlm.nih.gov/protein/NP_001341500.1)
**SEQ ID NO.: 2** (nucleic acid sequence of human Fetuin A)

```
ATGAAGTCCCTCGTCCTGCTCCTTTGTCTTGCTCAGCTCTGGGGCTGCCACTCAGCCCCACATGGCCC
AGGGCTGATTTATAGACAACCGAACTGCGATGATCCAGAAACTGAGGAAGCAGCTCTGGTGGCTATAG
ACTACATCAATCAAAACCTTCCTTGGGGATACAAACACACCTTGAACCAGATTGATGAAGTAAAGGTG
TGGCCTCAGCAGCCCTCCGGAGAGCTGTTTGAGATTGAAATAGACACCCTGGAAACCACCTGCCATGT
GCTGGACCCCACCCCTGTGGCAAGATGCAGCGTGAGGCAGCTGAAGGAGCATGCTGTCGAAGGAGACT
GTGATTTCCAGCTGTTGAAACTAGATGGCAAGTTTTCCGTGGTATACGCAAAATGTGATTCCAGTCCA
GCAGACTCAGCCGAGGACGTGCGCAAGGTGTGCCAAGACTGCCCCCTGCTGGCCCCGCTGAACGACAC
CAGGGTGGTGCACGCCGCGAAAGCTGCCCTGGCCGCCTTCAACGCTCAGAACAACGGCTCCAATTTTC
AGCTGGAGGAAATTTCCCGGGCTCAGCTTGTGCCCCTCCCACCTTCTACCTATGTGGAGTTTACAGTG
TCTGGCACTGACTGTGTTGCTAAAGAGGCCACAGAGGCAGCCAAGTGTAACCTGCTGGCAGAAAAGCA
ATATGGCTTTTGTAAGGCAACACTCAGTGAGAAGCTTGGTGGGGCAGAGGTTGCAGTGACCTGCATGG
TGTTCCAAACACAGCCCGTGAGCTCACAGCCCCAACCAGAAGGTGCCAATGAAGCAGTCCCCACACCC
GTGGTGGACCCAGATGCACCTCCGTCCCCTCCACTTGGCGCACCTGGACTCCCTCCAGCTGGCTCACC
CCCAGACTCCCATGTGTTACTGGCAGCTCCTCCAGGACACCAGTTGCACCGGGCGCACTACGACCTGC
GCCACACCTTCATGGGTGTGGTCTCATTGGGGTCACCCTCAGGAGAAGTGTCGCACCCCCGGAAAACA
CGCACAGTGGTGCAGCCTAGTGTTGGTGCTGCTGCTGGGCCAGTGGTTCCTCCATGTCCGGGGGAGGAT
CAGACACTTCAAGGTCTAG
```

Nucleotide Sequence: NM_001354571.2 (1107 nucleotides)
(https://www.ncbi.nlm.nih.gov/gene/197; NM_001354571.2 ; Consensus Coding Sequence CCDS87176.1).

In another preferred embodiment, the functionally active derivative or fragment of the naturally occurring protein Fetuin A is at least 70%, preferably at least 80%, more preferably at least 90%, even more preferably at least 95% or most preferably at least 99% identical and/or homologous to SEQ ID NO: 3 (bovine Fetuin A sequence), as defined below.

**SEQ ID** NO.: 3 (protein sequence of bovine Fetuin A)

```
MKSFVLLFCLAQLWGCHSIPLDPVAGYKEPACDDPDTEQAALAAVDYINKHLPRGYKHTLNQIDSVKV
WPRRPTGEVYDIEIDTLETTCHVLDPTPLANCSVRQQTQHAVEGDCDIHVLKQDGQFSVLFTKCDSSP
DSAEDVRKLCPDCPLLAPLNDSRVVHAVEVALATFNAESNGSYLQLVEISRAQFVPLPVSVSVEFAVA
ATDCIAKEVVDPTKCNLLAEKQYGFCKGSVIQKALGGEDVRVTCTLFQTQPVIPQPQPDGAEAEAPSA
VPDAAGPTPSAAGPPVASVVVGPSVVAVPLPLHRAHYDLRHTFSGVASVESSSGEAFHVGKTPIVGQP
SIPGGPVRLCPGRIRYFKI
```

Protein Sequence: NP_776409.1 (359 amino acids)
(https://www.ncbi.nlm.nih.gov/protein/NP_776409.1)
**SEQ ID NO.: 4** (nucleic acid sequence of bovine Fetuin A)

```
ATGAAATCCTTCGTTCTGCTCTTTTGCCTGGCTCAGCTCTGGGGCTGCCACTCGATCCCGCTTGACCC
GGTTGCAGGTTATAAGGAACCGGCCTGTGATGACCCAGACACAGAGCAAGCAGCCTTGGCTGCCGTGG
ACTACATCAACAAGCACCTTCCTCGGGGCTACAAGCACACCTTGAACCAGATTGACAGTGTGAAGGTG
TGGCCGAGGCGGCCCACGGGAGAGGTGTATGACATTGAAATAGATACCCTGGAAACCACCTGCCACGT
ACTGGACCCCACGCCCCTGGCGAACTGCAGCGTGAGGCAGCAGACGCAGCACGCGGTGGAAGGAGACT
GCGATATCCACGTGCTGAAACAAGATGGCCAGTTTTCCGTGCTGTTTACAAATGTGATTCCAGTCCA
GATTCCGCCGAGGACGTGCGCAAGTTGTGCCCAGACTGCCCCCTGCTGGCGCCACTCAACGACAGCCG
GGTGGTGCACGCAGTGGAGGTCGCGCTGGCTACCTTCAATGCCGAGAGCAACGGCTCCTACTTACAGC
TGGTGGAAATTTCTCGGGCTCAATTTGTGCCTCTTCCAGTTTCTGTCTCTGTGGAGTTTGCAGTGGCT
GCTACTGACTGTATTGCTAAAGAAGTCGTAGATCCAACCAAGTGCAACCTACTGGCAGAAAAGCAATA
TGGCTTCTGTAAGGGGTCAGTCATTCAGAAAGCTCTTGGTGGGGAGGACGTCAGAGTGACTTGCACGT
TGTTCCAAACGCAGCCTGTGATTCCGCAGCCCCAGCCCGACGGCGCCGAGGCTGAGGCCCCAAGCGCT
GTGCCGGACGCAGCTGGGCCTACGCCTTCTGCAGCTGGCCCGCCCGTGGCCTCCGTGGTGGTGGGGCC
AAGCGTGGTAGCAGTTCCCCTGCCGCTGCACCGAGCACACTACGACTTGCGCCACACTTTCTCCGGGG
TGGCCTCAGTGGAGTCATCCTCGGGAGAAGCGTTCCACGTGGGCAAAACACCCATAGTGGGGCAGCCT
AGCATTCCTGGAGGTCCAGTCCGCCTTTGCCCAGGGAGAATCAGATACTTCAAGATCTAG
```

Nucleotide Sequence: NM_173984.3 (Coding Nucleotide Sequence (1080 nt).
(https://www.ncbi.nlm.nih.gov/nuccore/NM_173984.3)

In another embodiment of the present invention, the Fetuin A is recombinantly expressed, preferably in a host cell system providing posttranslational modifications, more preferably glycosylations.

The term "expressed" as used herein generally means allowing or causing the information in a gene or a DNA sequence to become manifest. In particular, in the context of the present invention, it means the intracellular production of the protein encoded by the nucleic acid expression construct of the present invention by activating the cellular functions involved in transcription and translation of the corresponding gene or DNA sequence. That is, a DNA sequence is expressed in or by a cell to form an "expression product" such as a protein. The expression of a protein in or by a cell may depend on a variety of factors, including, but not limited to, the promoter sequence which is operably linked to the DNA sequence encoding the protein of interest, the presence or absence of enhancer and/or silencer sequences or other DNA sequences which control the rate of transcription, the cell culture conditions applied to the cell carrying the respective DNA sequence including the media used for culturing the cell, and/or the number of copies of DNA sequences introduced into the cell. Successful expression of the fusion protein may be analyzed and/or visualized by standard methods known to the person skilled in the art, including, but not limited to, Western Blot analysis, Northern Blot analysis, RNase protection assays, quantitative RT-PCR, as well as methods concerning direct or indirect fluorescence readout. "Recombinant" and "recombinantly" means artificially produced, relating to or denoting an organism, cell, protein or genetic material formed by recombination. Recombinant expression of desired protein is a standard method in the art, which is followed by standard protein purification methods as described below.

"Post-translational modifications" increase the functional diversity of the proteome by the covalent addition of functional groups or proteins, proteolytic cleavage of regulatory subunits or degradation of entire proteins. These modifications include, among others, phosphorylation, ubiquitination, S-nitrosylation, methylation, N-acetylation, lipidation and glycosylation.

A "host cell providing post-translational modifications" is a host cell, which delivers necessary modification modalities, enabling correct post-translational modification of mammalian proteins. Systems, which provide these requirements are for example insect cell expression systems, such as BTI-Tn5B1-4 or SF9 cell lines or mammalian cell expression systems, such as for example, but not restricted to those HEK 293 (T), CHO or HELA cells.

"Glycosylation" is defined as an enzyme-directed site-specific process that links saccharides to produce glycans, which in turn are attached to proteins, lipids or other organic molecules. Protein glycosylation as post-translational process is a natural process in cells by which saccharides are selectively added to specific protein residues in order to convey more structural stability or function to the native protein structure. Thereby, Glycosylation plays an important role in the proper functionality of a huge variety of proteins. Glycosylation includes, but is not limited to C-linked, N-linked, O-linked or S-linked glycosylation and glycation. "Glycation" thereby refers to the non-enzymatic attachment of reducing sugars to the nitrogen atoms of proteins.

An "injection" as used herein is a parenteral mode of administration, which does not encompass oral administration or inhalation. As used herein "injection" encompasses intravenous (iv), intraarterial, intramuscular (im), subcutaneous (sc) and intradermal (id), intraperitoneal, intraosseous, intracardiac, intraarticular and intracavernous administration. In some advantageous embodiments, injection refers to intravenous (iv) or subcutaneous (sc) administration. The injection may be a discontinuous and/or short-time injection, for example using a needle with a single dose of Fetuin A (and optionally injecting further doses at a later time). Alternatively, the injection may also be an infusion, i.e. a continuous injection for a longer time. In some embodiments an injection is administered within less than 15 min, in particular within less than 10 or within less than 5 min. Administration by injection also includes systemic administrations, for example administration via extracorporeal circulation (ECC). "Inhalation" as used herein describes the process of inhaling or breathing in, which may transport air, gases, active substances and aerosols into the lungs. For example, Fetuin A may be provided as part of an aerosol for the purpose of inhalation.

In some embodiments of the present invention, the Fetuin A is administered to the patient by way of injection or inhalation. In some preferred embodiments, Fetuin A is administered to the patient by way of injection. In some embodiments, Fetuin A is administered to the patient by way of intravenous (iv), intramuscular (im) or subcutaneous (sc) injection, preferably intravenous (iv) injection.

In some embodiments, the Fetuin A is administered to the patient in an amount effective for treating the renal disorder, in particular wherein the Fetuin A is administered to a patient in a concentration range from 1 mg/kg to 200 mg/kg of body weight, in particular administered intravenously. In some embodiments, Fetuin A is administered to a patient in a concentration range from 5 mg/kg to about 100 mg/kg of body weight, in particular administered intravenously. In some embodiments, Fetuin A is administered to a patient in a concentration range from 10 mg/kg to about 50 mg/kg of body weight, in particular administered intravenously. In some embodiments, Fetuin A is administered to a patient in a concentration range from 10 mg/kg to about 75 mg/kg of body weight, in particular administered intravenously. In some embodiments, Fetuin A is administered to a patient in a concentration range from 20 or 30 mg/kg to about 200 mg/kg of body weight, in particular administered intravenously. In some embodiments, Fetuin A is administered to a patient in a concentration range from 20 mg/kg to about 50 mg/kg of body weight, in particular administered intravenously. In some embodiments, Fetuin A is administered to a patient in a concentration range from 30 mg/kg to about 50 mg/kg of body weight, in particular administered intravenously.

In a further embodiment, the Fetuin A is administered to the subject in need thereof during major surgery, for example during cardiovascular surgery or during kidney transplantation. In a further embodiment, the Fetuin A is administered to the subject in need thereof after major surgery, for example after cardiovascular surgery or after kidney transplantation. In a further embodiment, the Fetuin A is administered to the subject in need thereof before major surgery, for example before cardiovascular surgery or before kidney transplantation. These embodiments may be combined, e.g. in some embodiments Fetuin A is administered before and after major surgery.

In the context of the present invention Fetuin A may be a target of the transcription factor HIF-1$\alpha$. HIF-1$\alpha$, also known as Hypoxia-inducible factor 1-alpha, is a subunit of a heterodimeric transcription factor hypoxia-inducible factor 1 (HIF-1). "Target" is herein defined as a protein, which is affected "downstream", relating to a cellular signaling pathway or cascade. In the context of the present invention, Fetuin A may act downstream of HIF-1$\alpha$.

In some embodiments of the present invention, the treatment comprises modulation of calcification levels in the kidney tissue, preferably hypoxia-related calcification levels. "Calcification" is hereby defined as the accumulation of calcium salts in a body tissue, in particular in the kidney that might lead to permanent damage of the tissue. It may lead to inflammatory response and tissue damage as a consequence of restricted oxygen supply in the tissue. In an embodiment, the treatment comprises removal of calcium mineral depositions in kidney tissue, in particular the removal of calcium mineral depositions in transplanted kidney tissue.

In another embodiment of the invention, at least two doses of the Fetuin A are administered or at least three doses of the Fetuin A are administered. In some embodiments, at least four doses of the Fetuin A are administered. In another embodiment of the invention the treatment of the subject comprises a treatment phase with at least daily or weekly administration of at least one dose of Fetuin A, preferably in the week or month after an ischemic incident and/or after surgery, in particular after major surgery.

In the context of the present invention the term "ischemic incident" refers to any event that causes and/or comprises ischemia, in particular that comprises ischemia. A surgery may be an ischemic incident when blood supply to tissue is restricted during said surgery. For example, during a kidney transplantation procedure the graft often cannot adequately be perfused with blood. A sudden blood loss during an accident can also be an ischemic incident during which the kidney is not adequately perfused. In some embodiments, the ischemic incident is ischemia in major surgery, in particular wherein the major surgery is kidney transplantation or cardiovascular surgery.

In another embodiment of the invention at least one dose of Fetuin A is administered prior, during or after an ischemic incident and/or surgery, in particular major surgery. In some embodiments the ischemic incident and/or the surgery is kidney transplantation or cardiovascular surgery.

In yet another embodiment of the invention, at least one dose of Fetuin A is administered within 48 hours, in particular within 24 hours, before or after an ischemic incident, such as kidney transplantation or cardiovascular surgery. In some embodiments, the at least one dose of Fetuin A is administered within 48 hours, in particular within 24 hours, after the ischemic incident. In yet another embodiment, at least one dose of Fetuin A is administered within 48 hours, in particular within 24 hours, before a major surgery. In yet another embodiment, at least one dose of Fetuin A is administered within 48 hours, in particular within 24 hours, after a major surgery. In some further embodiments, Fetuin A is administered during major surgery. Said major surgery is kidney transplantation or cardiovascular surgery in some embodiments.

In yet another embodiment of the invention, at least one first dose of Fetuin A is administered within 48 hours, in particular within 24 hours, before an ischemic incident and at least one second dose of Fetuin A is administered within 48 hours, in particular within 24 hours, after the ischemic incident. In some embodiments, the at least two doses of Fetuin A are administered within 48 hours, in particular within 24 hours, before an ischemic incident. In yet another embodiment, at least two doses of Fetuin A are administered within 48 hours, in particular within 24 hours, before a major surgery. In yet another embodiment, two or three doses of Fetuin A are administered within 48 hours, in particular within 24 hours, before a major surgery. In some embodiments, the at least two doses of Fetuin A are administered within 48 hours, in particular within 24 hours, after an ischemic incident. In yet another embodiment, at least two doses of Fetuin A are administered within 48 hours, in particular within 24 hours, after a major surgery. In yet another embodiment, two or three doses of Fetuin A are administered within 48 hours, in particular within 24 hours, after a major surgery.

In some embodiments, the Fetuin A is administered to the patient for a treatment period at least once weekly, in particular at least once every 48 hours, preferably at least once every 24 hours.

In some embodiments, Fetuin A is administered in divided doses over a total duration of 1 to 100 days, in particular over a total duration of 1 to 50 days. In another embodiment, Fetuin A is administered in divided doses over a total duration of 1 to 25 days. In another embodiment, Fetuin A is administered in divided doses over a total duration of 1 to 10 days. In another embodiment, Fetuin A is administered in divided doses over a total duration of 2 to 10 days.

In an embodiment of the present invention, the treatment comprises administering Fetuin A as part of a pharmaceutical composition to the subject in need thereof. Preferably, the pharmaceutical composition is in liquid form. Preferably, said pharmaceutical composition is in accordance with one or more of the embodiments as defined below in the second aspect of the present invention.

In a second aspect, the present invention relates to a pharmaceutical composition for use in treating a renal disorder, comprising Fetuin A (AHSG) and optionally at least one pharmaceutical acceptable carrier. In some embodiments, the second aspect relates to the pharmaceutical composition for use in a method for treating a renal disorder, wherein an amount of Fetuin A effective for treating the renal disorder is administered to a subject, preferably a patient, in need thereof.

A "pharmaceutical carrier" as referred to herein may be any substrate used in the process of drug delivery, which serves to improve the selectivity, effectiveness, and/or safety of drug administration.

The pharmaceutical composition in accordance with the second aspect may be for used for the same treatments and renal disorders as described above in relation to Fetuin A and any treatments described above also may describe embodiments of the pharmaceutical composition for use in such treatments. In a preferred embodiment of this second aspect, the renal disorder is selected from the group consisting of acute renal disorders, chronic renal disorders, kidney fibrosis, chronic kidney disease, renal insufficiency, renal inflammation, acute kidney injuries, ischemic renal disorders, disorders related to hypoxia, renal ischemia-reperfusion injury, kidney tissue damage, preferably ischemic kidney tissue damage, disorders related to kidney transplantation, disorders related to cardiovascular surgery and combinations thereof.

In some embodiments, the pharmaceutical composition is a liquid. In some embodiments, the pharmaceutical composition comprises water.

In another embodiment of this second aspect, the disorders related to kidney transplantation are selected from the group consisting of delayed graft function, organ rejection of kidney transplants, kidney tissue damage resulting from kidney transplantation, inflammation resulting from kidney transplantation, renal IRI resulting from kidney transplantation and combinations thereof.

Preferably, the renal disorder is caused by hypoxia during surgery, in particular major surgery. For example, the hypoxia may be caused by ischemia during kidney transplantation or cardiovascular surgery.

In equally preferred embodiments, the Fetuin A of the pharmaceutical composition is defined as described in detail above. The pharmaceutical composition may comprise any of the above-described embodiments of Fetuin A.

In the context of the pharmaceutical composition of the present invention, Fetuin A is preferably human Fetuin A, more preferably Fetuin A, which is derived from human blood plasma.

In an alternative embodiment the Fetuin A of the pharmaceutical composition is recombinantly expressed, preferably in a host cell system providing posttranslational modifications, in particular glycosylation.

Equally preferred in this context is that the Fetuin A is at least 70%, at least 80%, at least 90%, at least 95% or at least 99% identical and/or homologous to SEQ ID NO: 1. Also preferred is that the Fetuin A comprises or consists of SEQ ID NO: 1 or fragments thereof.

In an embodiment, the pharmaceutical composition is administered to a subject as described in detail above. Preferably, the pharmaceutical composition is formulated for injection or inhalation.

The following Figures and Examples are intended to illustrate various embodiments of the present invention. As such, the specific modifications discussed therein are not to be understood as limitations of the scope of the invention as defined by the claims.

## FIGURES

### Figure 1

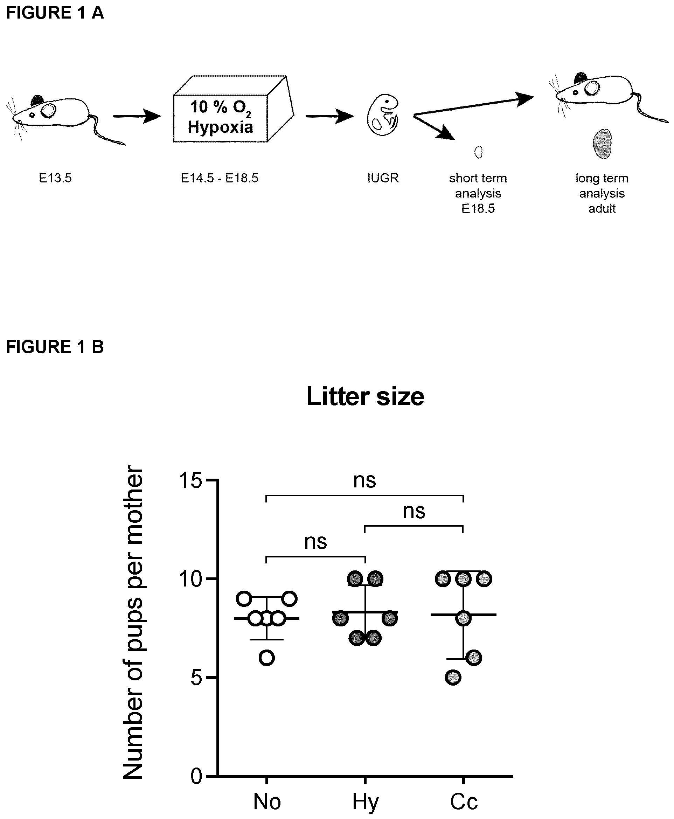

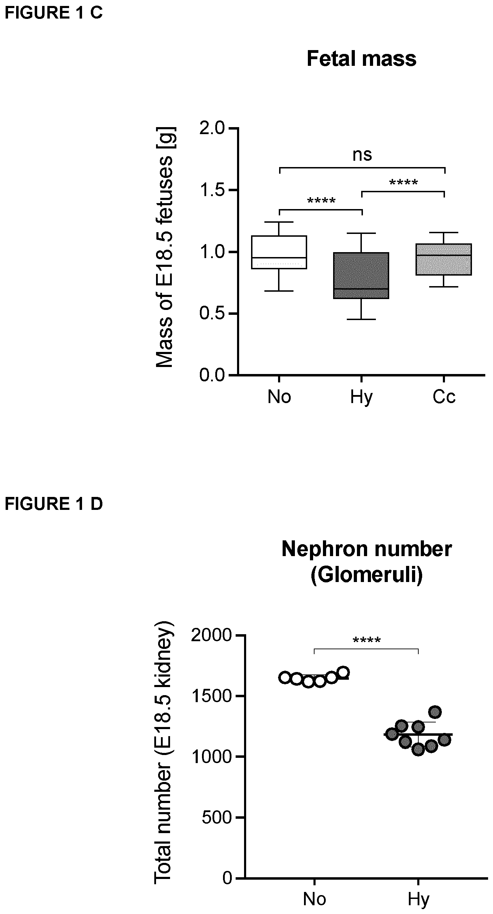

Chronic fetal hypoxia induces intra-uterine growth restriction in mice: **Figure 1 A.** Experimental setup and time points of analysis. **Figure 1 B.** Mean number of pups per litter: **Figure 1 C.** Total weight distribution of E18.5 fetuses (n=49 for each experimental condition; No: normoxic, Hy: hypoxic, Cc: caloric controls). **Figure 1 D.** The mean number of nephrons per E18.5 kidney was determined by staining for the glomerular marker nephrin. Unpaired t test with Welch's correction.

### Figure 2

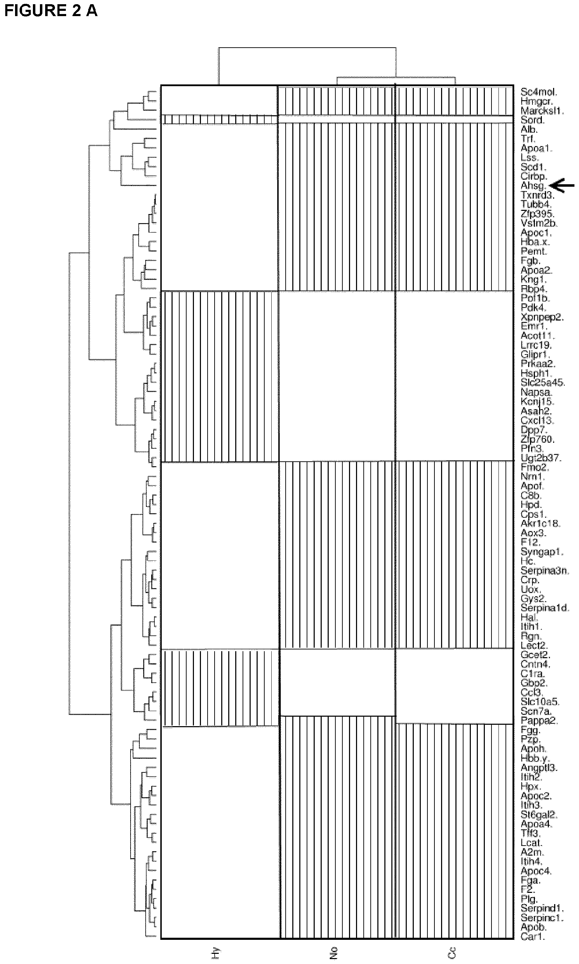

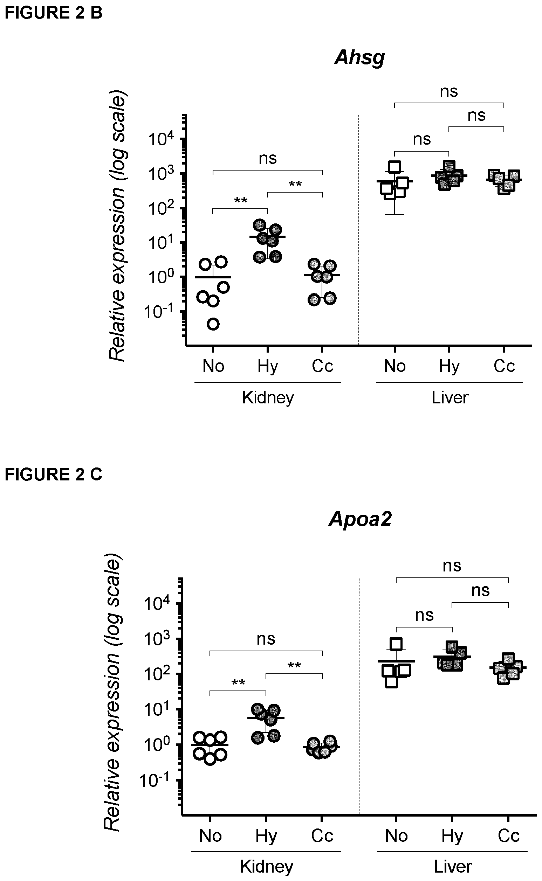

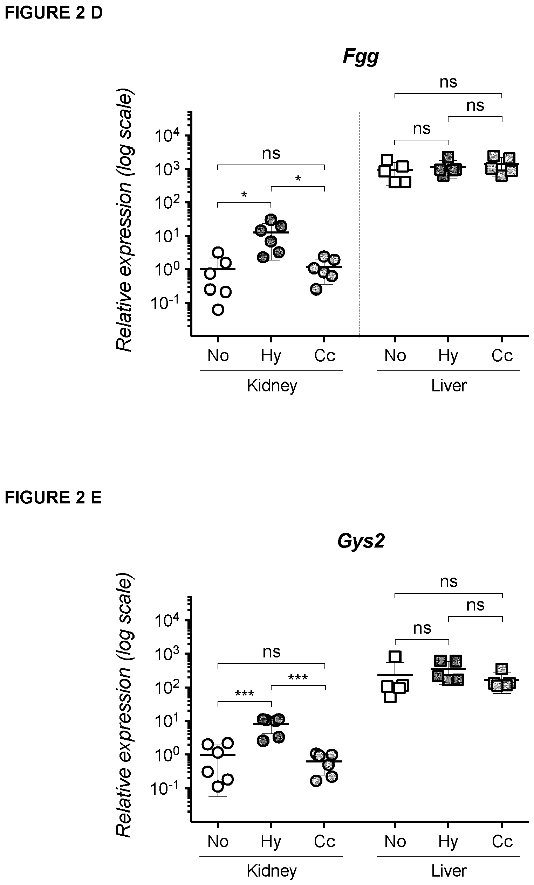

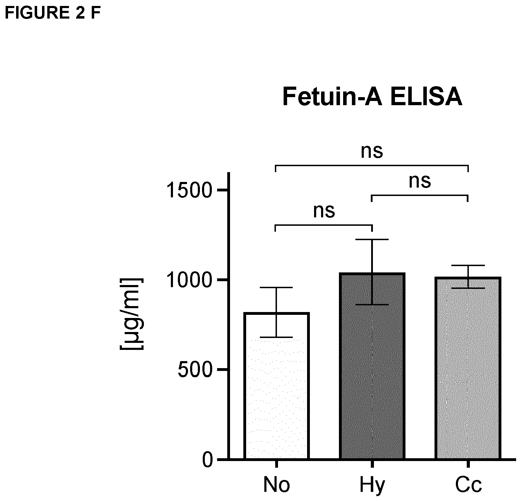

Hypoxia-induced gene expression in the kidney: **Figure 2 A.** Hierarchical clustering of cDNA microarray data comparing the renal gene expression profiles of hypoxic, normoxic caloric control group E18.5 fetuses (n=3 per experimental condition). White regions indicate induction and vertically hatched region repression (degree of induction, repression not indicated in Figure 2 A). The position of *Ahsg* is marked by an arrow. Clustering was performed for genes with at least 1.3-fold regulation of hypoxic vs. both normoxic controls; 1way ANOVA; P<0.05. Figure 2 B-E. Relative mRNA values of *Ahsg* (**B**), *Apoa2* (C), *Fgg* (**D**) and *Gys2* (**E**) in E18.5 kidneys (circles) or liver samples (squares). **Figure 2 F.** Fetuin A plasma levels of E18.5 fetuses assessed by ELISA.

### Figure 3

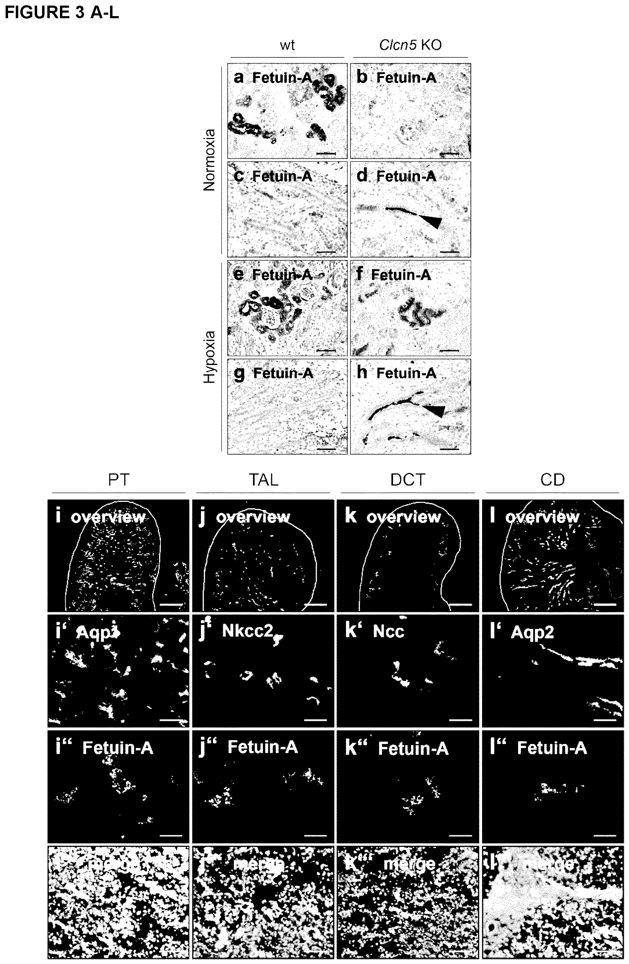

Fetal hypoxia induced Fetuin A expression in the proximal tubulus: Figure 3 A-H. Fetuin A staining on E18.5 kidney sections of normoxic (**A-D**) or hypoxic (**E-H**), wildtype **(A,C,E,G)** or *Clcn5* KO **(B,D,F,H)** fetuses. Arrowheads in (**D,H**) indicate intraluminal Fetuin A staining that results from the impaired endocytosis of low molecular weight proteins in the PT of *Clcn5* KO mice. (**I-L**) Immunofluorescence staining of the indicated nephron segment marker proteins and Fetuin A on E18.5 kidney sections. PT, proximal tubulus; TAL, thick ascending limb; DCT, distal convoluted tubulus; CD, collecting duct; Scale bars, 300 $\mu$m (overview images) or 50 $\mu$m.

### Figure 4

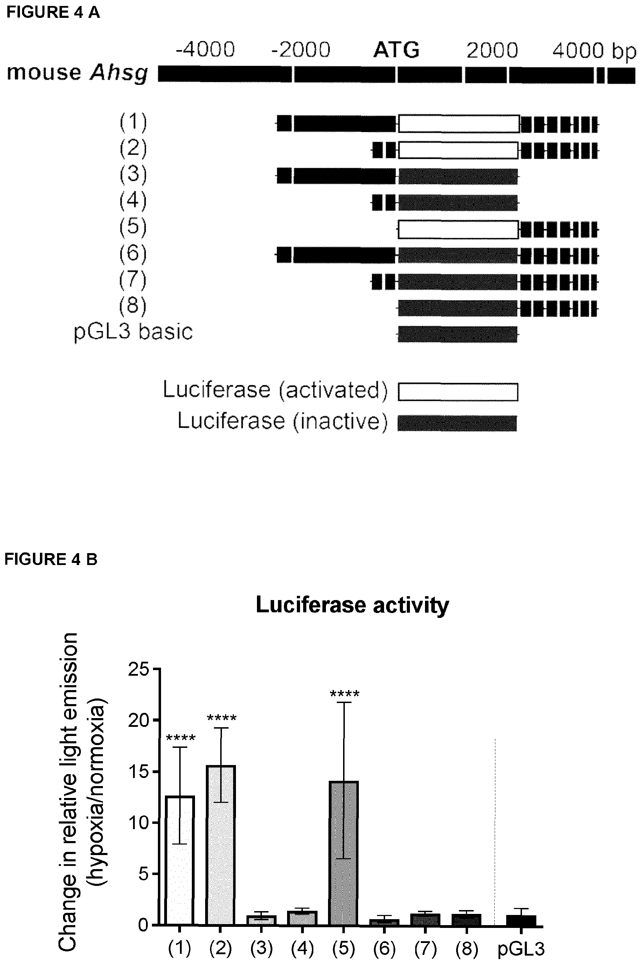

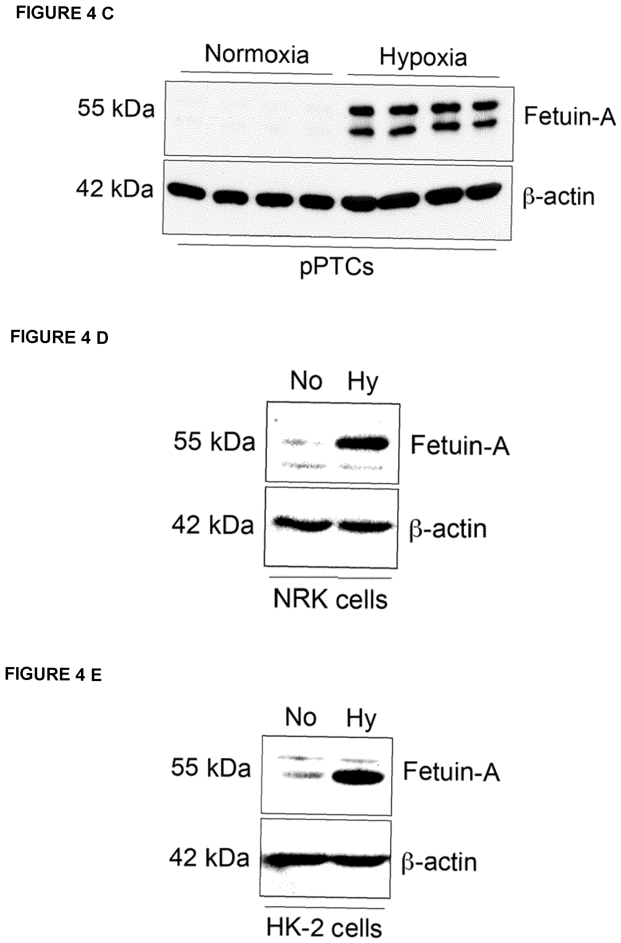

Hypoxia activated Fetuin A expression *in vitro:* **Figure 4 A.** Depiction of the potential HREs of mouse *Ahsg* that were used to generate the luciferase reporter gene constructs (1-8). Mutated HREs are shown. **Figure 4 B.** Luciferase activity in NRK cells transfected with the reporter constructs. The fold change in luciferase light emission between hypoxic and normoxic culture conditions is shown. Each condition is normalized to the empty vector control (pGL3). Dunnett's multiple comparisons test. Figure 4 C-E. Expression of Fetuin A in mouse primary proximal tubular cells (pPTC) isolated from 4 different mice (C), in the rat cell line NRK (**D**) and in the human cell line HK-2 (**E**) cultured under normoxic or hypoxic conditions.

### Figure 5

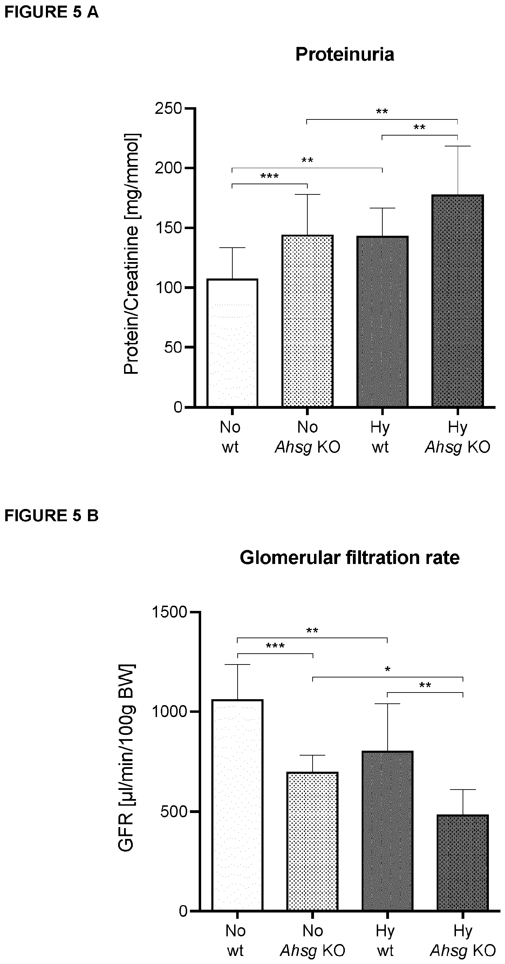

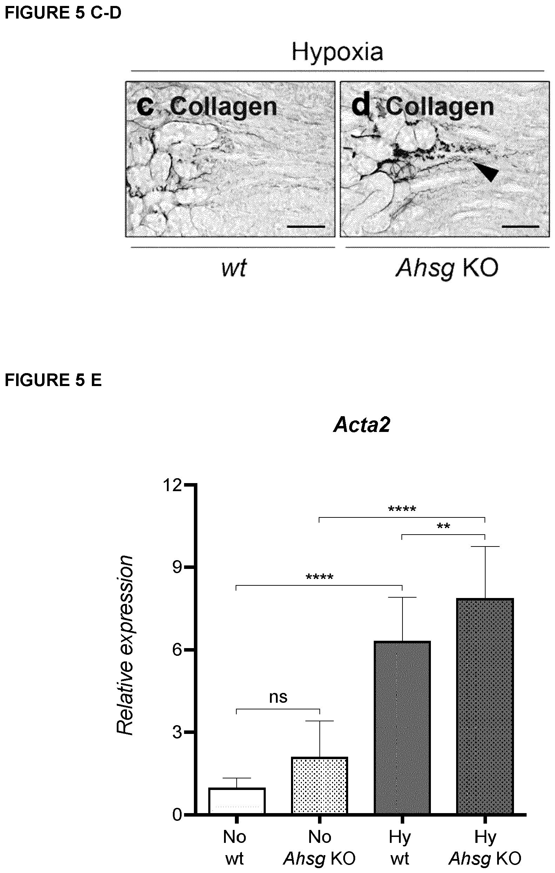

Fetuin A deficiency aggravated CKD progression in hypoxic IUGR kidneys: Figure 5 A-B. The decline in kidney function of 9 week old mice as assessed by the protein/creatinine ratio and the glomerular filtration rate (GFR). **Figure 5 B.** is most pronounced fetal hypoxic *Ahsg* KO animals, showing an additive effect of hypoxia and Fetuin A deficiency (n≥5 for each experimental condition). Figure 5 C-D. Representative image of picrosirius red staining for collagen shows a stronger, more intricate pattern on kidney sections derived from P14 fetal hypoxic *Ahsg* KO mice (**D**) compard to fetal hypoxic wt mice (**C**). Figure 5 E-G. The relative expression levels of the fibrosis markers *Acta2* (**E**), *Col1a1* (**F**) and *Fn1* (**G**) are markedly enhanced in kidneys of fetal hypoxic *Ahsg* KO mice (n≥5 for each experimental condition). Fisher's LSD test (**A-B**). Scale bars, 50 $\mu$m.

### Figure 6

Fetuin A deficiency promoted accumulation of calcium mineral particles and macrophages in hypoxic IUGR kidneys:

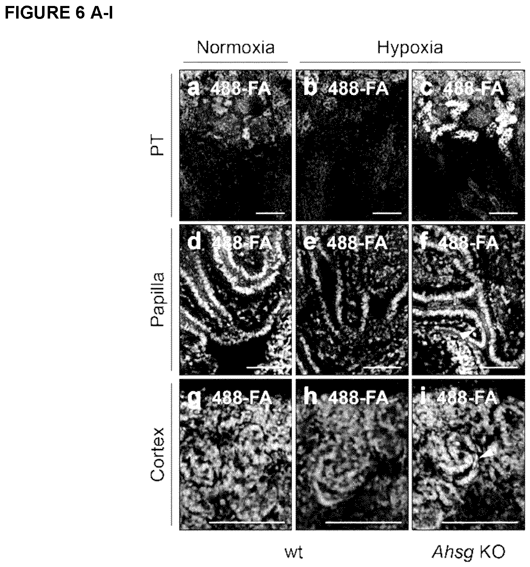

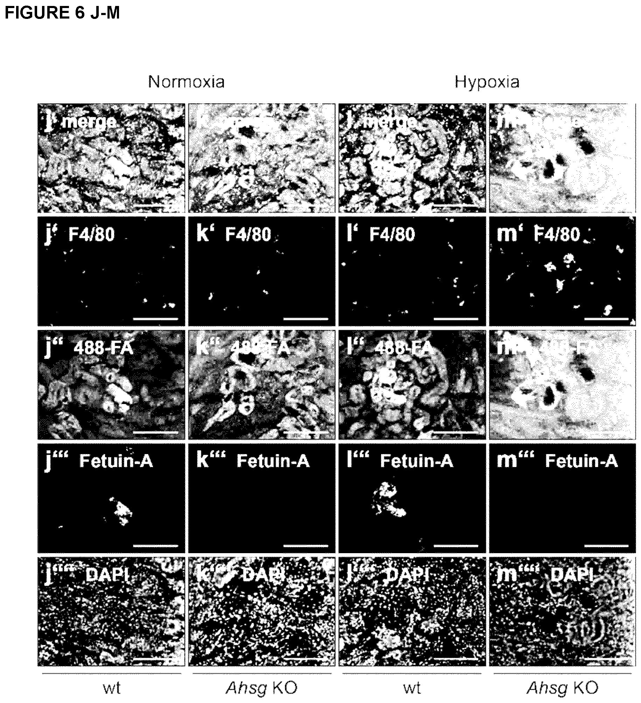

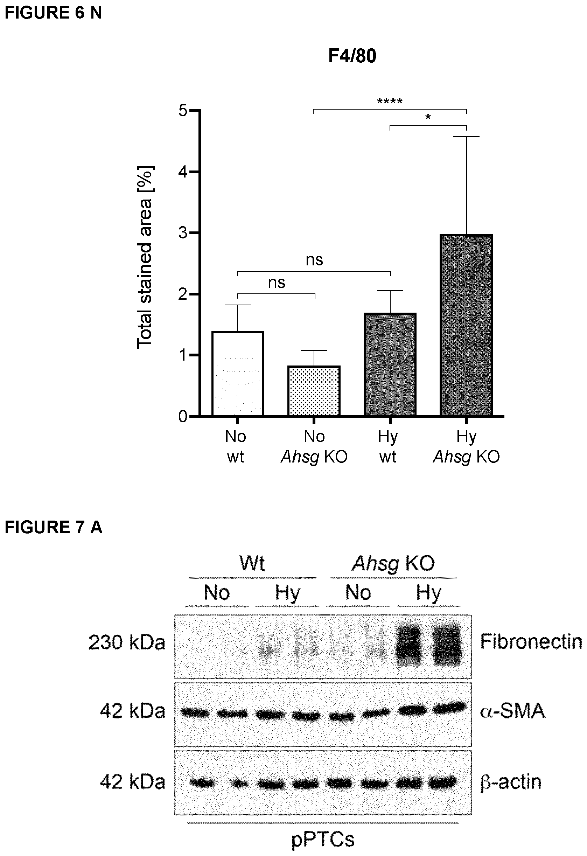

Figure 6 A-I. Detection of calcium biominerals by ATTO 488 fluorescently labelled Fetuin A (488-FA) staining on E18.5 kidney sections of normoxic wt (**A,D,G**), hypoxic wt (**B,E,H**) or hypoxic *Ahsg* KO embryos (**C,F,I**). Hypoxic *Ahsg* KO mice exhibit the strongest staining intensity in the PT (**C**) compared to normoxic and hypoxic wt mice (**A,B**) indicative of an increased mineralized matrix turnover. The arrowheads point towards granular staining pattern, reflecting bulk accumulation of 488-FA in the papilla (**F**) and cortex (**I**) only present in hypoxic *Ahsg* KO mice. Figure 6 J-M. Representative immunofluorescence staining for the macrophage marker F4/80 (j', k', l', m')and Fetuin A (j''', k''', l''', m'''), counterstained with DAPI (j'''', k'''', l'''', m'''') and 488-FA (j'', k'', l'', m'') on E18.5 kidney sections for the indicated genotypes and oxygen conditions. An accumulation of macrophages and diffuse as well as granular 488-FA staining is only detected in hypoxic *Ahsg* KO mice (**M**). **Figure 6 N.** Quantification of total F4/80 stained area per field of view. Multiple sections of at least 3 mice were analysed for each experimental condition. Scale bars, 100 μm.

## Figure 7

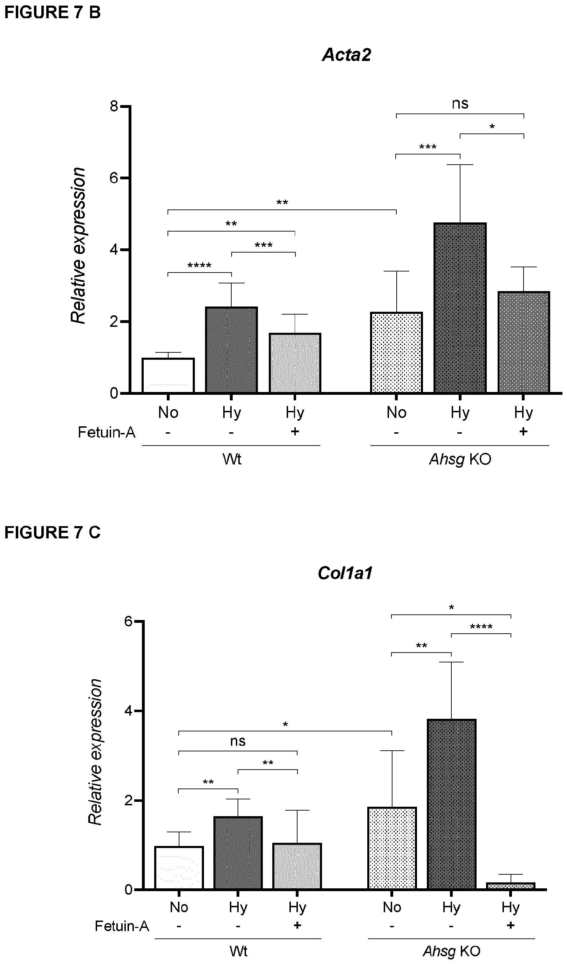

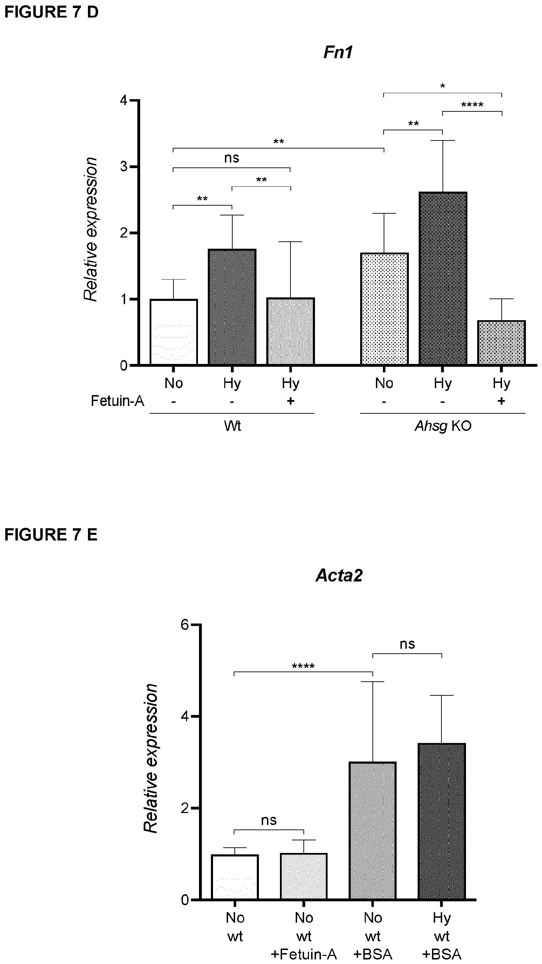

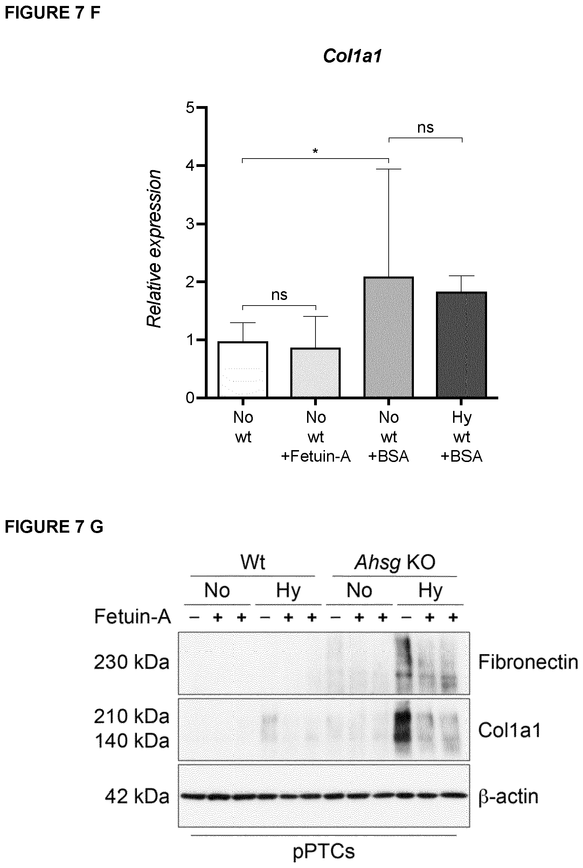

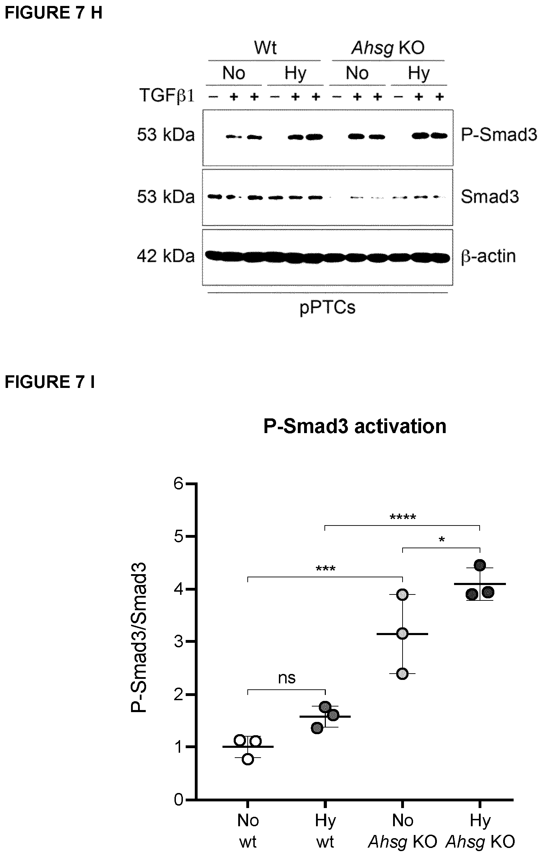

Fetuin A attenuated hypoxia-induced expression of fibrotic markers: **Figure 7 A.** Representative Western blot showing the induction of fibronectin and α-smooth muscle actin (α-SMA) upon hypoxic culture conditions in primary proximal tubular cells (pPTCs) isolated from wt or *Ahsg* KO mice. Figure 7 B-D. Fetuin A supplementation attenuates the hypoxia-induced expression of the fibrotic markers *Acta2* (**B**), *Col1a1* (**C**) and *Fn1* (**D**) in pPTCs (n≥5 for each experimental condition). Figure 7 E-F. Only Fetuin A, but not BSA, has a diminishing effect on the expression of fibrotic markers (n≥4 for each experimental condition). **Figure 7 G.** Representative Western blot depicting that Fetuin A supplementation reduces the expression of fibronectin and collagen type I (Col1a1) protein. **Figure 7 H.** TGF-β1 treatment and hypoxia have an additive effect on the phosphorylation of Smad3. A representative Western blot is shown. **Figure 7 I.** Quantification of Smad3 activation shown in (**H**). Unpaired t test with Welch's correction (**B-D**, only for comparison of normoxic wt and normoxic *Ahsg* KO samples), Fisher's LSD test (**I**).

## Figure 8

Hypoxia, resp IRI induced deposition of calcium containing microparticles in the kidney: Detection of calcium containing microparticles with 488-FA following ischemia reperfusion (IRP). 488-FA staining on control side (l) and ischemia reperfusion (m) 7d after surgery shows the presence of calcium biominerals only in IRP kidneys.

## Figure 9

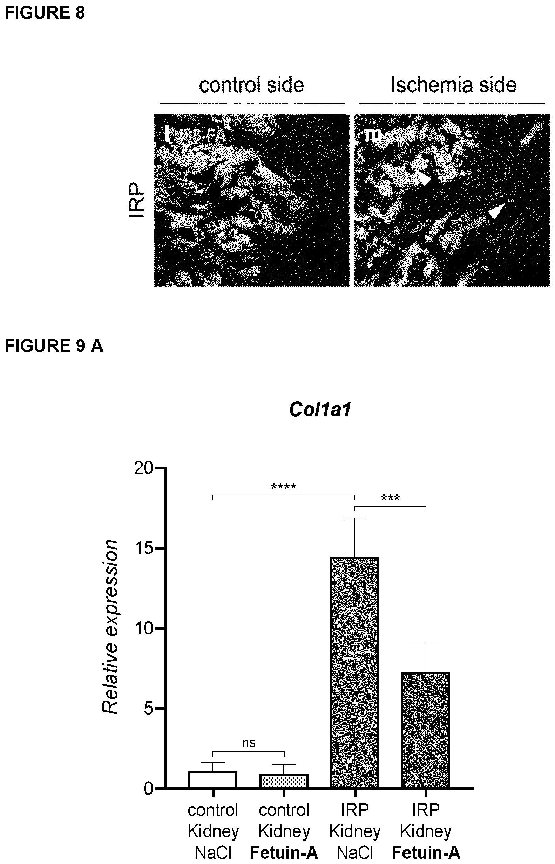

Fetuin A supplementation attenuated the expression of fibrotic markers following ischemic reperfusion injury: Figure 9 A-F. Fetuin A supplementation attenuates ischemia-induced expression of *Col1a1* (**A**), *Col3a1* (**B**), Co16a1 (**C**), but has no effect on the expression of the non-collagen fibrotic markers *Acta2* (**D**), *Fn1* (**E**) or *Vim* (**F**). One-way ANOVA test.

## Figure 10

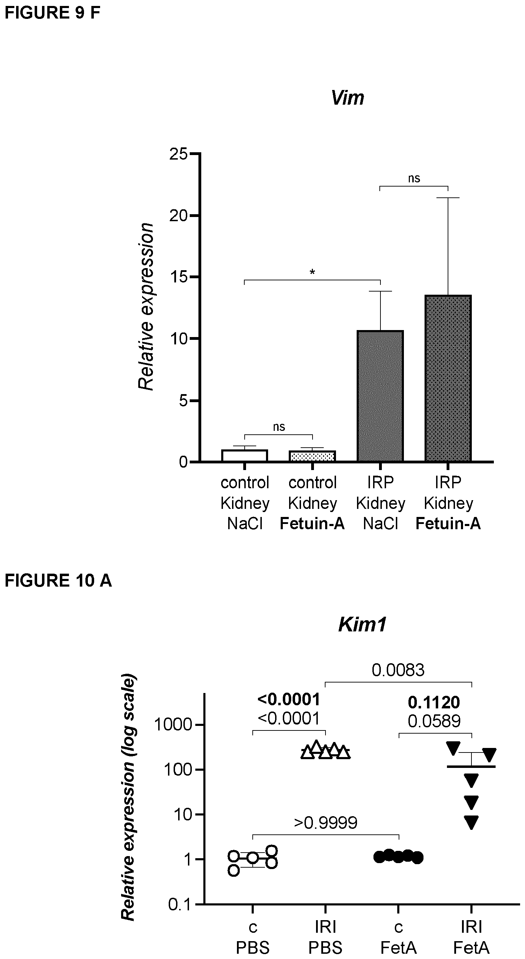

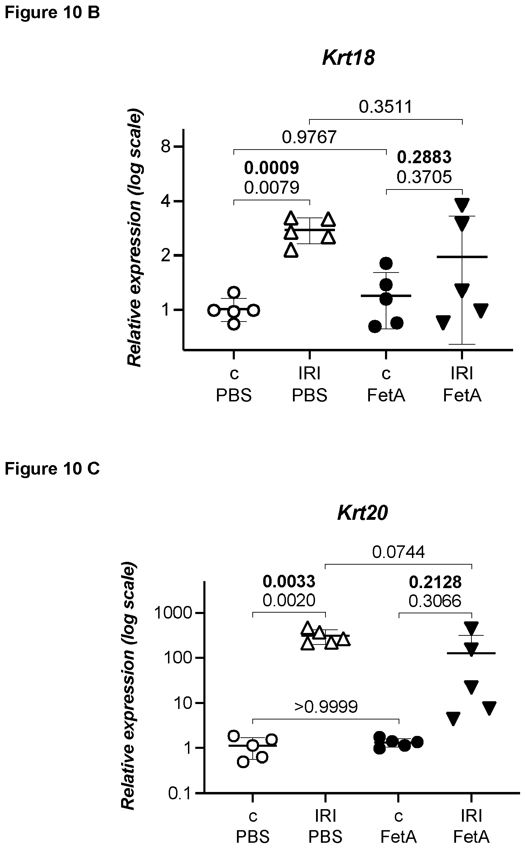

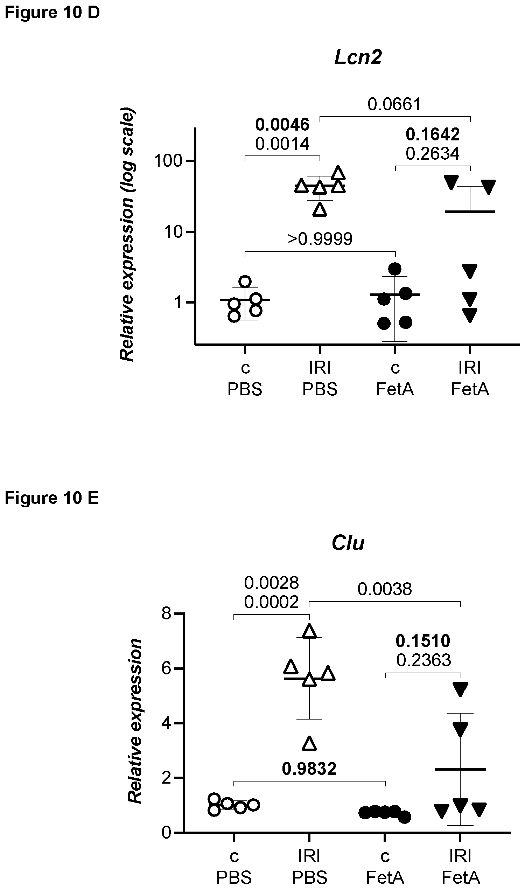

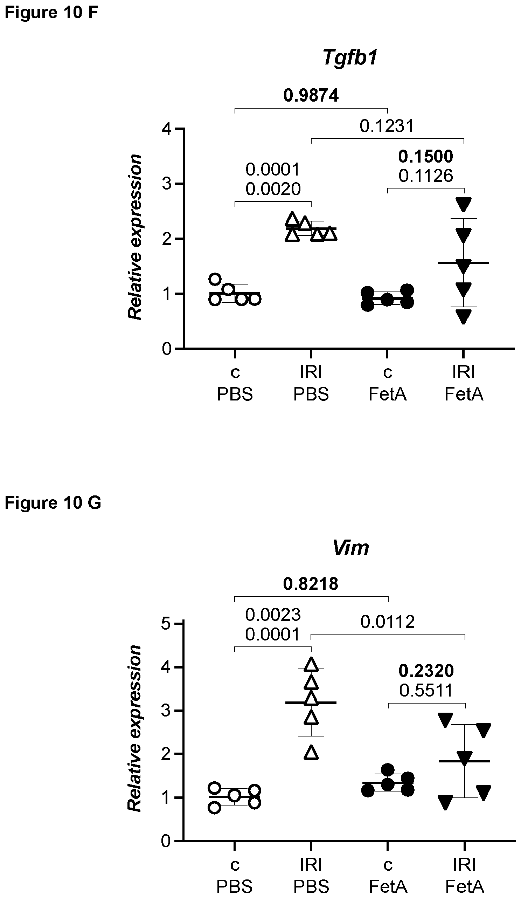

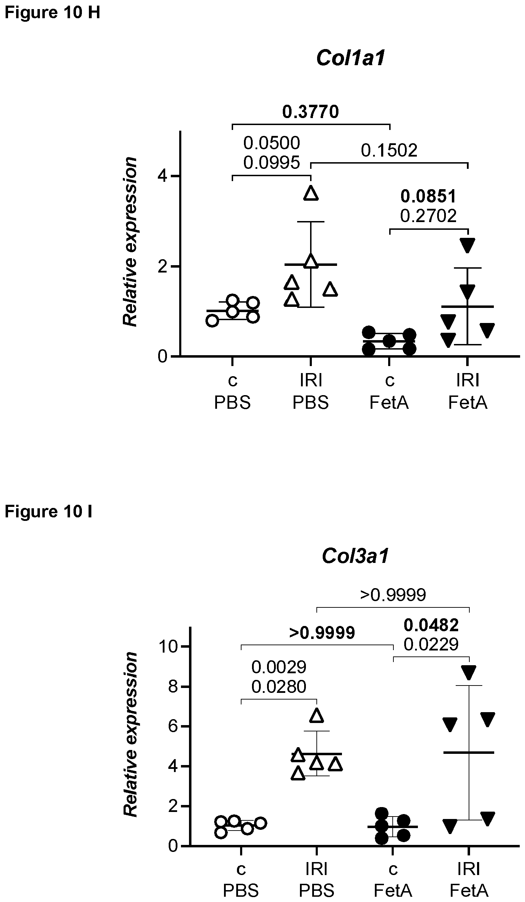

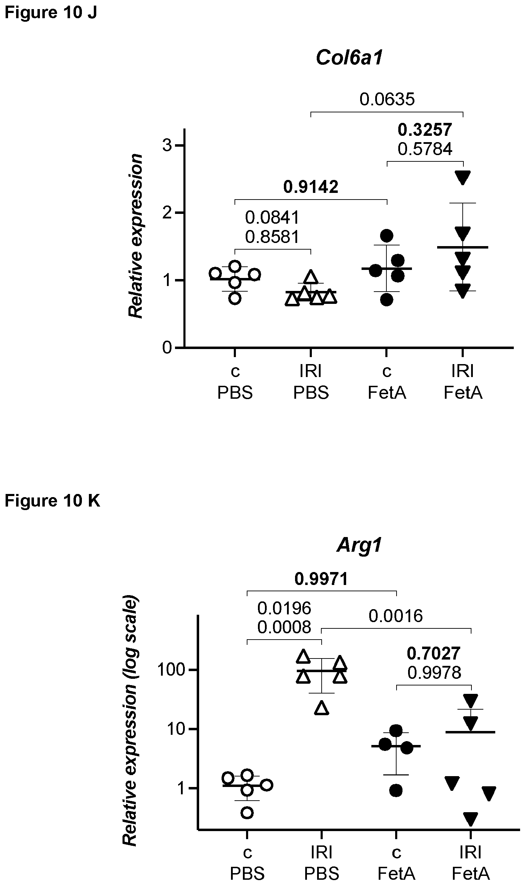

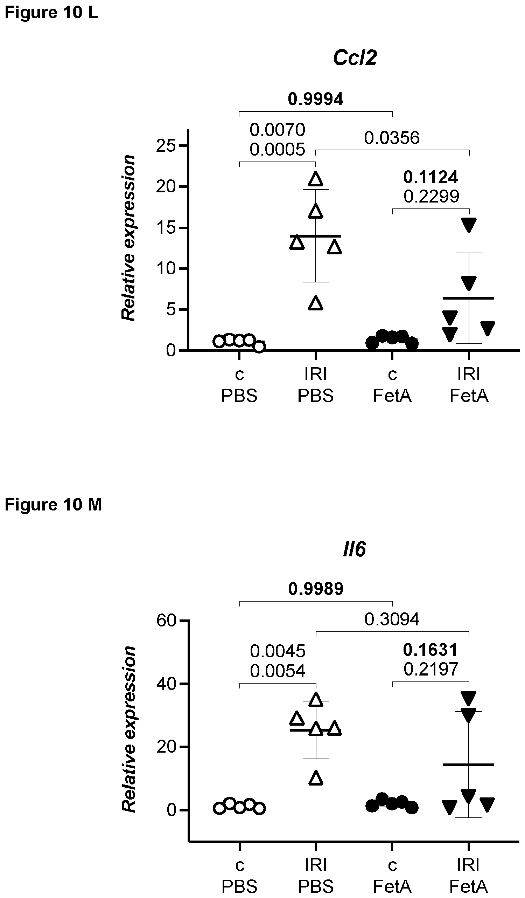

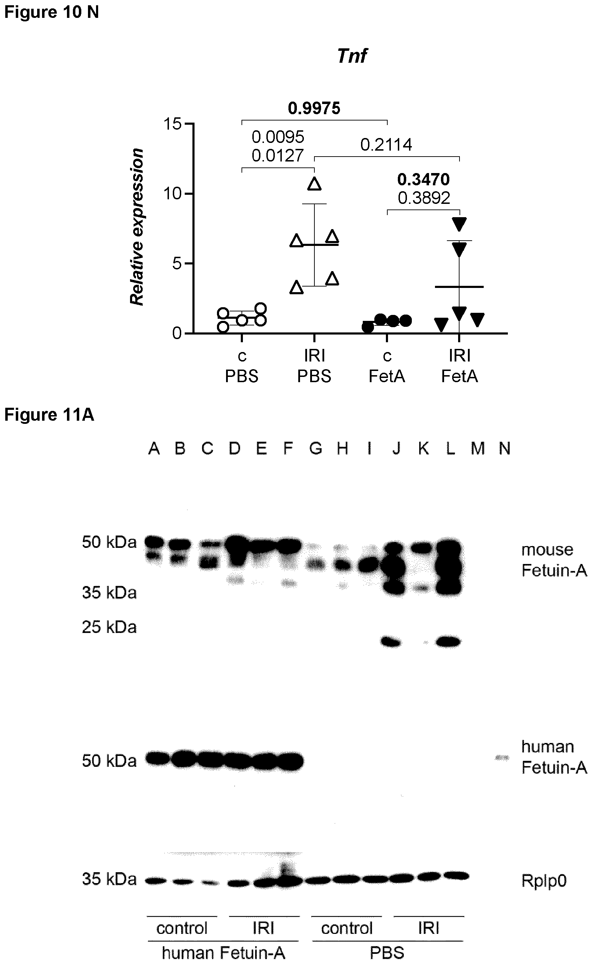

Prophylactic administration of human Fetuin A in mice undergoing ischemia reperfusion injury (IRI) led to less damage and less fibrotic remodeling: Figure 10 A-D. Expression levels of early damage markers *Kim1* (**A**), *Krt18* (**B**), *Krt20* (**C**) and *Lcn2* (**D**) are reduced in IRI kidneys pretreated with Fetuin A compared to IRI kidneys pretreated with PBS. **Figure 10 E and F.** Fibrotic remodeling markers *Clu* (**E**) and *Tgfb1* (**F**) are reduced as well compared to PBS control during IRI. **Figure 10 G.** The non-collagenous fibrotic marker *Vim* (**G**) is reduced in Fetuin A pretreated IRI kidneys compared to PBS pretreated IRI kidneys. Figure 10 H-J. Collagen expression upon Fetuin A prophylactic treatment is shown for *Col1a1* (**H**), *Col3a1* (**I**) and *Co16a1* (**J**). **Figure 10 K.** *Arg1* (**K**) expression is repressed in Fetuin A pretreated mice compared in PBS pretreated mice, indicating a reduced requirement for collagen synthesis. Figure 10 L-N. Pretreatment with Fetuin A also reduces the expression of chemokines *Ccl2* (**L**), *Il-6* (**M**) and *Tnf-α* (**N**) involved in damage signaling and repair that are usually increased under ischemic conditions. Legend for Figure 10: *open circles*: PBS injected, unoperated control kidneys; *open triangles*: PBS injected, IRI kidneys; *filled black circles*: Fetuin A injected, unoperated control kidneys; *filled black triangles*: Fetuin A injected, IRI kidneys. P values in Figure 10 are denoted for ordinary one-way ANOVA with Tukey correction for multiple comparisons (regular font) and paired t test (bold font).

## Figure 11

Presence of exogenous Fetuin A in the renal tissue of mice upon intravenous administration:

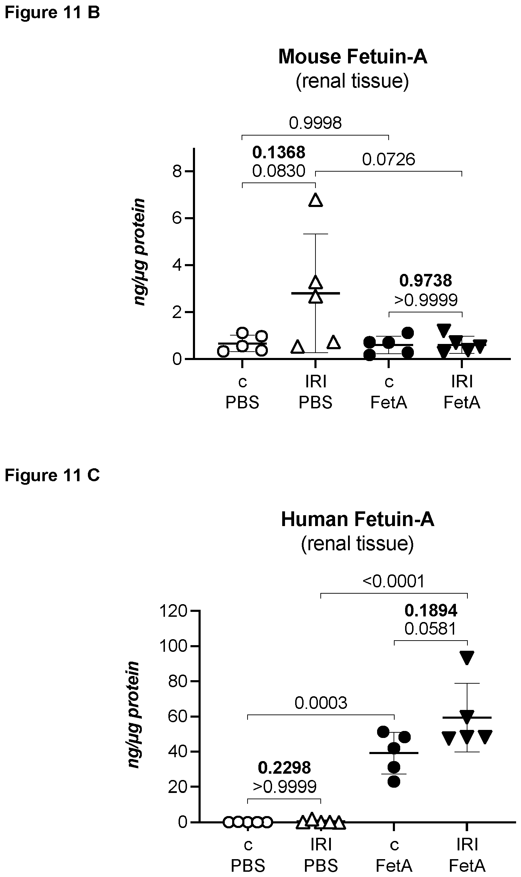

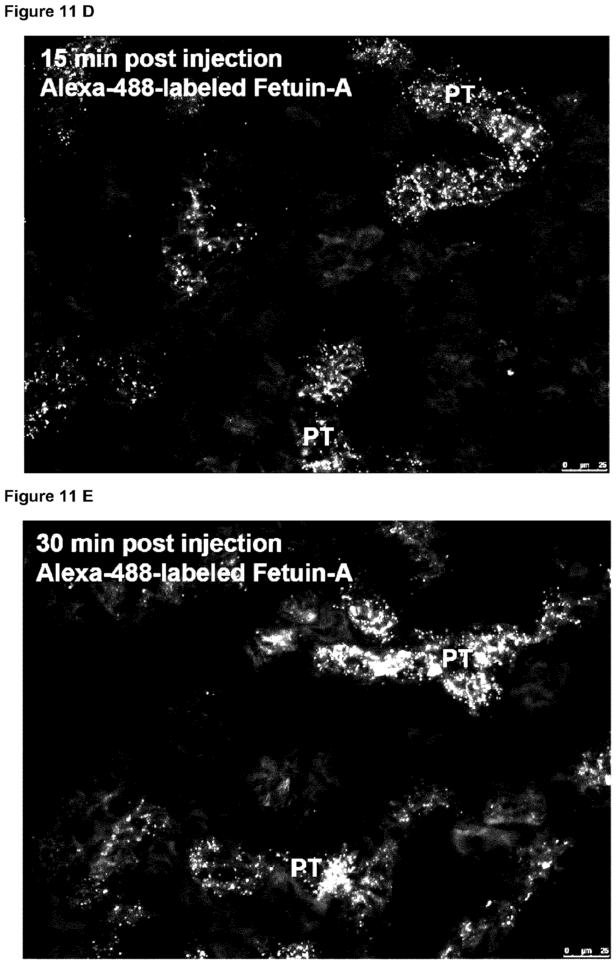

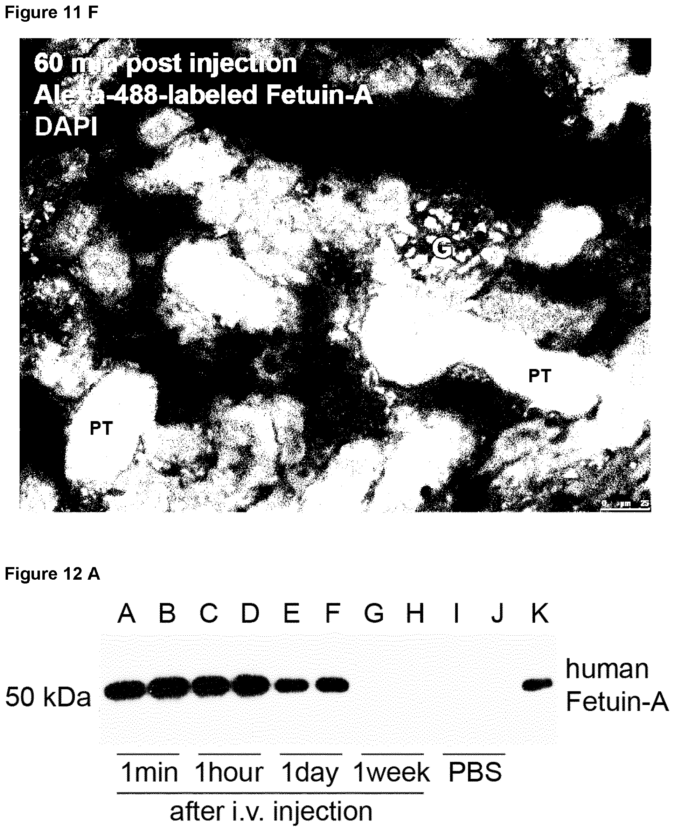

**Figure 11 A.** Western blots. Lanes A to C: Fetuin A injected, unoperated control kidneys; Lanes D to F: Fetuin A injected, IRI kidneys; Lanes G to I: PBS injected, unoperated control kidneys; Lanes J to L: PBS injected, IRI kidneys; Lane M: empty; Lane N: human Fetuin A protein used for injection. Top panel: Western blot for endogenous mouse Fetuin A using the ab187051 anti-Fetuin A antibody (specific for mouse Fetuin A) from abcam. Middle panel: Western blot for human Fetuin A using the sc-133146-HRP anti-Fetuin A antibody (specific for human Fetuin A) from Santa Cruz. Bottom panel: Rplp0 served as loading control. Kidney samples from mice injected with human Fetuin A protein showed a strong band for human Fetuin A (lanes A-F). No Fetuin A was detected in PBS-injected control samples (lanes G-L). PBS-injected IRI kidneys (lanes J-L) showed the highest abundance of mouse Fetuin A among all samples. Human Fetuin A was not detected with the mouse-specific anti-Fetuin A antibody ab187051, because human Fetuin A protein (lane N) gave no signal. **Figure 11 B.** ELISA for mouse Fetuin-A using the MFTA00 ELISA kit (specific for mouse Fetuin-A) from bio-techne. Endogenous mouse Fetuin A was only elevated in PBS-injected IRI kidneys compared to all other samples. **Figure 11 C.** ELISA for human Fetuin A using the DFTA00 ELISA kit (specific for human Fetuin-A) from bio-techne. Human Fetuin A could not be detected in kidney samples of PBS-injected mice. Fetuin A injected IRI kidneys have higher tissue levels of human Fetuin A than the unoperated contralateral (right) kidney. Figure 11 D-F. Exogenous Fetuin A was present in the renal tissue of mice upon intravenous administration. Fluorescently labeled Fetuin A (Alexa-488-labeled Fetuin A) as present in the proximal tubules (PT) 15, 30, and 60 minutes after intravenous injection. 15 minutes after injection of Alexa-488-labeled Fetuin A, a fine punctated staining was detected in the proximal tubules. The intensity of this staining increased at 30 minutes and at 60 minutes after injection. G, glomerulus. Legend for Figure 11 B and C: *open circles*: PBS injected, unoperated control kidneys; *open triangles*: PBS injected, IRI kidneys; *filled black circles*: Fetuin A injected, unoperated control kidneys; *filled black triangles*: Fetuin A injected, IRI kidneys. P values in Figure 11 B and C are denoted for ordinary one-way ANOVA with Tukey correction for multiple comparisons (regular font) and paired t test (bold font).

### Figure 12

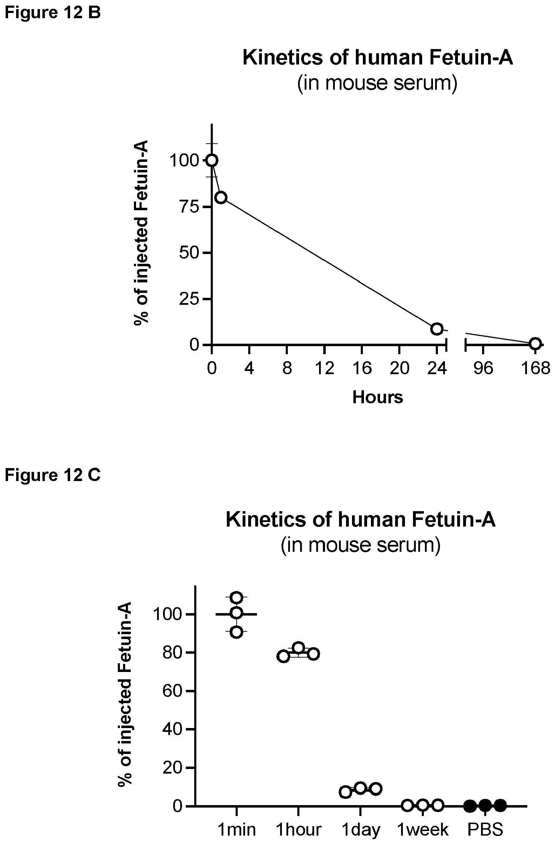

Time course of human Fetuin A in the serum of mice upon intravenous administration: **Figure 12 A.** Lanes A and B: 1 minute after injection; lanes C and D: 1 hour after injection; lanes E and F: 1 day after injection; lanes G and H: 1 week after injection; lanes I and J: control PBS injection; lane K: human Fetuin A protein used for injection. Western blot for human Fetuin A using the human-specific sc-133146-HRP anti-Fetuin A antibody from Santa Cruz. After 1 day, the detected amount of human Fetuin A in the serum was reduced compared to 1 minute or 1 hour post injection. After 1 week no human Fetuin A was detectable. Mouse Fetuin A was not detected with the sc-133146-HRP anti-Fetuin A antibody, because PBS-injected control animals give no signals. **Figure 12 B.** Normalized ELISA data using the DFTA00 ELISA kit (specific for human Fetuin-A) from bio-techne, showed the decline of intravenous injected human Fetuin A in mouse serum samples. Compared to the injection time point (1min), the amount of circulating human Fetuin A was reduced to 80 % after 1 hour, and to 8.5 % after 1 day. After 1 week, human Fetuin A could not be detected anymore, similar to PBS-injected mice (black circles). **Figure 12 C.** Time course of human Fetuin A in the serum of mice upon intravenous administration. Normalized ELISA data showing the decline of intravenous injected human Fetuin A in mouse serum samples. Compared to the injection time point (1min), the amount of circulating human Fetuin A was reduced to 80 % after 1 hour, and to 8.5 % after 1 day. After 1 week, human Fetuin A could not be detected anymore.

## EXAMPLES

**Chronic fetal hypoxia induces IUGR in mice.** To model chronic fetal hypoxia, timed-mated pregnant mice were exposed to 10% oxygen from E14.5 to E18.5 (FIGURE 1 A). As it was observed that hypoxic dams (Hy) ate less despite free food access and since caloric restriction itself is a known inducer of IUGR5, a second control group was included in the analysis to exclude the possibility that the findings might not be due to hypoxia, but rather to reduced ingested calories. In this caloric control (Cc) group, normoxic dams were fed with an amount of food matching the reduced amount of food consumed by the hypoxic mice. Litter size, placental mass or fetal viability were indistinguishable among the three groups, whereas maternal weight gain was significantly reduced in hypoxic dams (FIGURE 1 B). Importantly, only fetuses from hypoxic dams showed LBW, fulfilling IUGR criteria (FIGURE 1 C), whereas fetuses from the normoxic or the Cc group did not. Kidneys of hypoxic fetuses were smaller with significantly fewer nephrons compared to controls (FIGURE 1 D).

**Hypoxic fetal kidneys adopt a hepatic gene expression pattern.** Next, a whole genome expression in fetal hypoxic kidneys was examined using gene arrays. 62 induced and 28 repressed genes were identified and compared to both control groups (FIGURE 2 A). Of the induced genes, 17 are known to be regulated by hypoxia, including the bona fide HIF target genes transferrin, trefoil factor 3, neuritin, alpha-1- antitrypsin (Serpina1d) and alpha-1-antichymotrypsin (Serpina3n). Furthermore, more than 20% of the induced genes were found to be frequently purified from calciprotein particles (CPPs), comprising the major CPP components Fetuin A (Ahsg), albumin, Apo-A1 and thrombin (F2). Functional annotation clustering of the induced genes revealed in hypoxic kidneys an enrichment of secreted plasma proteins that are normally transcribed exclusively by the liver. Validation of the microarray data by RT-qPCR of select genes confirmed a more than 10- fold induction only in hypoxic fetal kidneys, but not in control group kidneys (FIGURES 2 B-E). No change in mRNA levels were found in fetal livers samples.

**Fetuin A is produced locally in the proximal tubulus under hypoxic conditions.** Interestingly, the gene with the highest induction (Ahsg) was found in 7 of the 10 annotation groups. Ahsg belongs to the cystatin superfamily of cysteine protease inhibitors, encoding for the negative acute phase glycoprotein Fetuin A, whose main function concerns mineralized matrix metabolism. Despite its strong induction in hypoxic kidneys, no significant rise could be detected in fetal plasma Fetuin A levels (FIGURE 2 F), implying a specific, local, rather than a systemic functional relevance during hypoxic developmental challenges. To further address the functional relevance of Ahsg expression in fetal hypoxic kidneys, its precise localization was examined. Figure 3 shows immunohistochemistry of fetal kidney proximal tubules (FIGURES 3 A, B, E, F) or tubule lumen (FIGURE 3 C, D, G, H). Strong Fetuin A staining demarcated cells of the proximal tubule (PT) regardless of the oxygen conditions (FIGURES 3 A, E). This is due to filtration and uptake of systemic Fetuin A into the PT via megalindependent endocytosis, masking any Fetuin A locally produced in the hypoxic fetal kidney. To selectively visualize Fetuin A protein of renal origin, a genetic approach was employed to block endocytosis into the PT, an alternative method to the 7 pharmacological inhibition of megalindependent endocytosis using His-sRAP (histidinetagged soluble receptor-associated protein). Clcn5 knock-out (KO) mice show severely impaired endocytosis of low molecular weight proteins in the PT, mimicking Dent's disease. Normoxic Clcn5 KO kidneys lacked the prominent Fetuin A staining in PT cells (FIGURE 3 B). Instead, a strong intraluminal signal was detected (FIGURE 3 D), which was not present in wildtype (wt) samples (FIGURE 3 C), highlighting the impaired endocytic phenotype of Clcn5 KO mice. However, hypoxic Clcn5 KO kidneys showed strong Fetuin A staining in the PT in addition to the luminal signal (FIGURE 3 F, H), providing evidence that the observed cellular Fetuin A staining genuinely originated in the PT. Double immunofluorescence staining for Fetuin A and different renal segment markers (FIGURE 3 I-L) confirmed that Fetuin A was expressed only in the PT of hypoxic fetal kidneys. Whole mount in situ hybridization corroborated Fetuin A mRNA synthesis in hypoxic fetal kidneys, but not in normoxic kidneys.

**Ahsg harbours putative HIF binding sites overlapping with enhancer regions.** Having shown that Fetuin A is locally produced in hypoxic fetal kidneys, it was assessed whether the expression of Ahsg was directly activated by hypoxia. To check for potential HIF binding sites (hypoxia response elements - HRE) in the human AHSG locus, HIF-1α (HIF-1-alpha) and HIF-2α (HIF-2-alpha) ChIP-seq datasets were used derived from hypoxic MCF7 cells. A cluster of potential HREs near exon 4 of human AHSG that overlapped with H3K27Ac and H3K4Me1 (chromatin marks of active enhancer elements) and DNaseI hypersensitivity was identified. Another putative HRE was located in intron 1. Screening Ahsg genes of 15 species for the presence of the consensus HIF binding sites (RCGTG) 10kb upand downstream of the ATG revealed a peak 1-5kb downstream of the ATG with an average number of 2 HREs per 1kb window. Notably, not only the annotated human ChIP-seq HIF sites localized within this peak, but also four potential mouse HREs. Alignment of the latter with enhancer marks revealed a close association with H3K27Ac, H3K4Me1 and DNaseI hypersensitivity.

**Hypoxia activates Fetuin A transcription in vitro.** Five putative HREs of mouse Ahsg and their surrounding DNA, alongside with nonsense mutations of these sites, were cloned into luciferase reporter plasmids (FIGURE 4 A). Normal rat kidney epithelial (NRK) cells transfected with reporter plasmids containing only the putative -2kb HRE did not show increased luciferase activity under hypoxic conditions (FIGURE 4 B). Conversely, NRK cells carrying reporter plasmids containing the downstream HREs significantly increased luminescence in hypoxia. No increased luciferase activity was detected when these HREs were mutated. Furthermore, there was no enhanced luciferase activity when up- and downstream HREs were combined. In summary, the HREs located downstream of the ATG confer hypoxia inducibility to the mouse Ahsg gene. Lastly, Fetuin A protein in primary mouse PT cells (pPTCs), NRK cells and human HK2 cells was detected when cultured under hypoxic conditions (FIGURE 4 C-E). Taken together, these findings identified Fetuin A as a novel, evolutionary conserved HIF-dependent target gene.

**Fetuin A deficiency aggravates CKD progression in hypoxic IUGR kidneys.** To investigate how the induction of Fetuin A in fetal hypoxic IUGR kidneys affects renal function in the long term, urinary protein levels were measured and the glomerular filtration rate (GFR) was determined in adult mice (FIGURES 5 A,B). GFR was reduced and proteinuria was increased in 9 weeks old mice exposed to fetal hypoxia vs. normoxia. Furthermore, assessment of fibrotic tissue remodelling revealed more collagen structures extending deeper into subcortical regions and the highest expression levels of fibrotic markers in kidneys of 9 fetal hypoxic Ahsg KO mice compared to hypoxic wt and normoxic controls, respectively (FIGURES 5 C-G). Importantly, these results clearly show that renal function was systematically more affected in Ahsg KO mice, showing an additive effect of hypoxia and Fetuin A deficiency.

**Fetuin A deficiency promotes accumulation of calcium mineral particles and macrophages in hypoxic IUGR kidneys.** Adult Fetuin A KO mice are prone to soft tissue calcification, however overt calcification in the kidneys of hypoxic Ahsg KO mice as assessed by von-Kossa staining was not detected. To still test whether the expression of Fetuin A in fetal hypoxic kidneys affected mineralized matrix handling for the presence of calcium containing nanoparticles was probed by incubating freshly cut kidney sections of E18.5 embryos with ATTO 488 fluorescently labelled Fetuin A (488-FA). Due to the high affinity binding of Fetuin A to calcium phosphate, 488-FA staining is more sensitive to detect calcium containing matrix and cell remnants than the commonly used mineral staining protocols. Thus, positive 488-FA staining in the absence of von-Kossa or Alizarin-Red staining also highlights structures merely enriched with calcium, including amorphous calcium-phosphate aggregates that often precede overt calcifications. 488-FA staining revealed in normoxic wt kidneys intense labelling of the PT, a site of major calcium resorption and thus also of mineralized matrix handling (FIGURE 6A). PT staining intensity was reduced in hypoxic wt and increased in hypoxic Ahsg KO, suggesting increased and reduced mineralized matrix turnover, respectively (FIGURES 6 B, C). Only hypoxic Ahsg KO kidneys also showed a granular staining pattern in the papillary region and less frequently in the nephrogenic region of the outer cortex (arrowheads in FIGURE 6 D-I), indicating bulk mineral deposition in the absence of endogenous Fetuin A. Excess bulk mineral or cellular debris is often found at sites of enhanced cell death. Indeed, TUNEL staining confirmed apoptosis in hypoxic Ahsg KO kidneys, but not in normoxic or hypoxic wt kidneys. Cleaved caspase-3 immunostaining in hypoxic Ahsg KO kidneys further corroborated cell death in these kidneys. Lastly, accumulation of mineralized material triggers an inflammatory response, leading to macrophage infiltration, tissue damage and fibrosis. Staining for the macrophage marker F4/80 revealed a significant accumulation of these cells only in hypoxic Ahsg KO vs. wt kidneys, suggesting a protective effect of Fetuin A (FIGURES 6 J-N). Collectively, these data illustrate for the first time the role of Fetuin A in binding and clearance of mineralized matrix in the kidney, protecting tissue integrity.

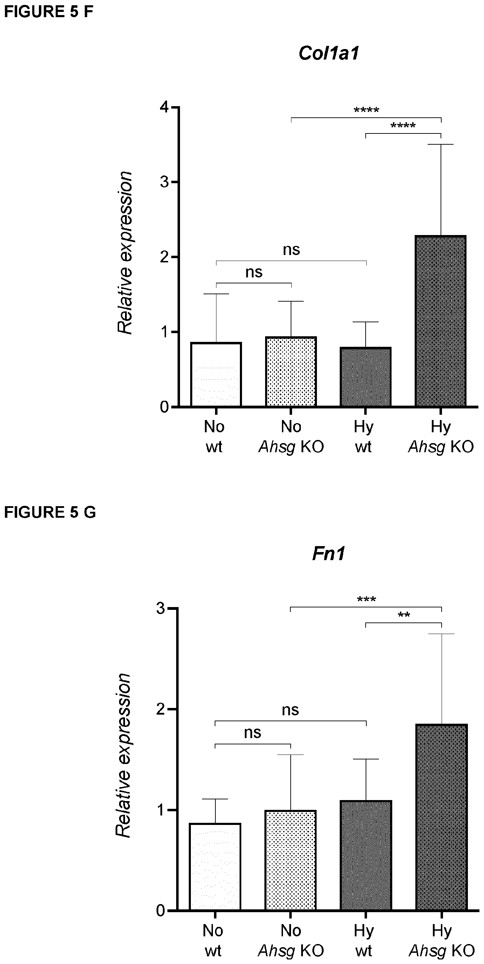

**Fetuin A attenuates the hypoxia-induced expression of fibrosis markers by antagonising TGF-β signaling.** Kidneys of fetal hypoxic Ahsg KO mice generally exhibited higher expression levels of fibrotic markers vs. fetal hypoxic wt animals (FIGURE 5), despite similar mRNA levels of transforming growth factor beta-1 (Tgfb1), a potent inducer of fibrosis. These findings were elucidated in vitro, using freshly isolated pPTCs. The expression of fibrotic markers was generally enhanced in Ahsg KO pPTCs compared to wt cells and even further increased under hypoxic culture conditions (FIGURES 7 A-D, G). This hypoxia-induced increase was blunted by supplementation of Fetuin A to the culture medium (FIGURES 7 B-G). Stimulation of pPTCs with recombinant TGF-β1 resulted in robust phosphorylation of its intracellular signal transducer Smad3, which was more than three times stronger in Ahsg KO pPTCs vs. wt cells (FIGURE 7 H, I), showing that Ahsg KO cells responded more vividly to TGF-β1. In contrast, adding Fetuin A before TGF-β1 treatment decreased Smad3 phosphorylation. These findings are in line with previous reports, describing Fetuin A as a TGF-β type II receptor mimic and cytokine antagonist. Collectively, the results suggest that Fetuin A reduces hypoxia- 11 induced renal fibrosis by direct antagonization of TGF-β1 signalling, in addition to its protective role as a mineral chaperone.

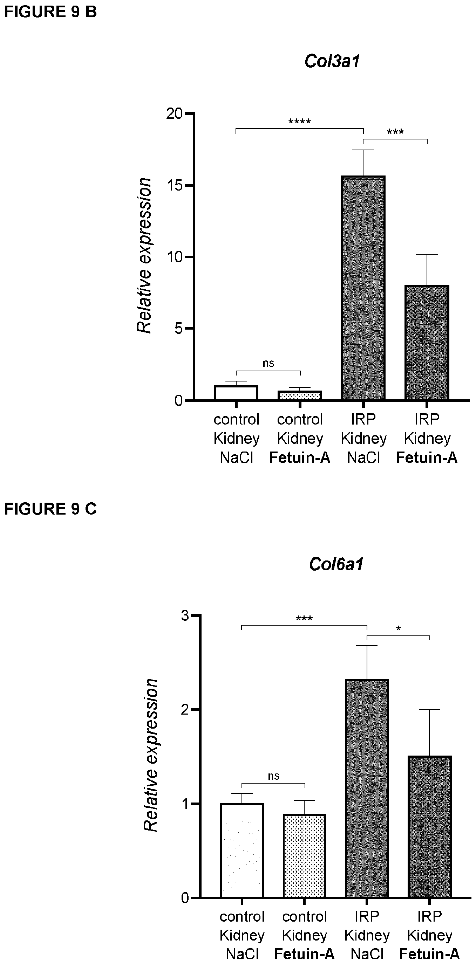

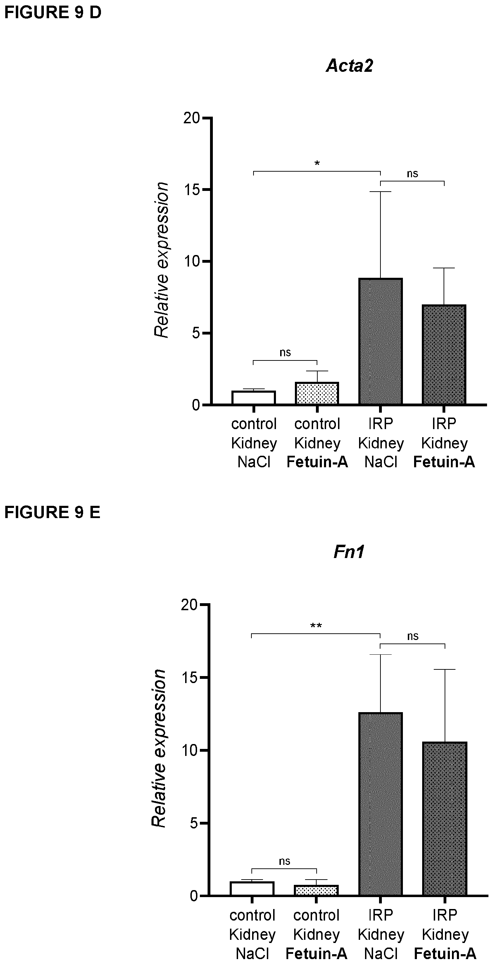

**Fetuin A significantly reduces in vivo the expression of fibrosis markers in the kidney after ischemia reperfusion injury.** Next, the therapeutic potential of Fetuin A supplementation on the treatment of kidney injury was experimentally explored. To that purpose, a well-established mouse model of unilateral ischemia-reperfusion injury was used. This IRI model, as indicated by its name, best mimicks ischemia-reperfusion lesions in the kidney following cardiovascular surgery, kidney transplant or removal of renal tumors. It is straightforward and reproducible. Ischemia was induced in the left kidney by clamping the renal vessels for 30 min, the right kidney served as a control. It was first shown by accumulation of calcium mineral particles and macrophages in hypoxic IUGR kidneys that ischemia-reperfusion indeed induces deposition of calcium mineral nanoparticles in the operated kidney but not in the contralateral control kidney (FIGURE 8). Additionally, treatment of IRI mice by Fetuin A vs. NaCl 0.9% showed that already at day 5, Fetuin A treatment significantly reduces specific fibrosis markers (Collagens *Col1a1, Col3a1* and Co16a1) in the ischemic, injured kidney compared to animals treated with NaCl 0.9% (FIGURE 9 A, B, C). In contrast, Fetuin A supplementation has no effect on the expression of the non-collagen fibrotic markers *Acta2, Fn1* or *Vim* (FIGURE 9 D, E, F). Therefore, Fetuin A supplementation attenuates the expression of fibrotic markers following ischemic reperfusion injury. In summary, these in vivo experiments clearly establish the role of Fetuin A as mineral chaperone safeguarding the kidney from hypoxic, resp. IR injuries.

**Prophylactic administration of human Fetuin A in mice undergoing ischemia reperfusion injury (IRI) led to less tissue damage and reduced fibrotic remodeling.** 900 μg Fetuin A were injected intravenously 24 h and again 3 h before unilateral IRI of the left kidney (20 min ischemia time). PBS was injected in control animals. Kidneys were collected 24 h post operation, the right kidney served as control. Expression levels of early damage markers *Kim1* (Figure 10 A), *Krt18* (Figure 10 B), *Krt20* (Figure 10 C) and *Lcn2* (Figure 10 D) were shown to be reduced in IRI kidneys pretreated with Fetuin A. This already shows that pretreatment with Fetuin A alleviated the tissue damage of reperfusion injury (IRI). Additionally, the observed reduction in *Vim* expression (Figure 10 G) indicated less epithelial-to-mesenchymal transition (EMT). Collagenous remodeling is a relatively late event during fibrotic remodeling, which does not play an important role 24 hours after IRI and, consequently, *Col1a1* (Figure 10 H), *Col3a1* (Figure 10 I) and Co16a1 (Figure 10 J) did not show an unexpected change. *Arg1* (Figure 10 K) expression was repressed in Fetuin A pretreated mice, indicating a reduced requirement for collagen synthesis. *Arg1* is important for increased synthesis of collagens, since it is required for the supply of L-proline, a main component of collagens. Moreover, pretreatment with Fetuin A reduced the expression of chemokines *Ccl2* (Figure 10 L), *Il-6* (Figure 10 M) and *Tnf-α* (Figure 10 N) involved in damage signaling and repair. In conclusion these experiments, in particular the early damage markers, show that prophylactic administration of human Fetuin A in mice undergoing ischemia reperfusion injury (IRI) leads to less damage and reduced fibrotic remodeling even at relatively low concentrations of Fetuin A.

**Presence of exogenous Fetuin A in the renal tissue of mice after intravenous (iv) administration.** No human Fetuin A was detected in PBS-injected samples (Figure 11 A, lanes G-L), confirming the specificity of the sc-133146-HRP anti-Fetuin A antibody for human Fetuin A and not mouse Fetuin A. PBS-injected IRI kidneys (Figure 11 A, lanes J-L) showed the highest abundance of mouse Fetuin A among all samples, indicative of a high degree of biomineralization and tissue damage in these kidneys. Kidney samples from mice injected with human Fetuin A protein showed a strong band for human Fetuin A (Figure 11 A, lanes A-F), confirming that Fetuin A was present in the renal tissue after intravenous administration. Endogenous mouse Fetuin A was only elevated in PBS-injected IRI kidneys compared to all other samples, indicative of a high degree of biomineralization and tissue damage in these kidneys (Figure 11 B). Human Fetuin A was not detected in PBS-injected kidney samples, confirming the specificity for human Fetuin A and not mouse Fetuin A of the DFTA00 ELISA kit from R&D systems (bio-techne) (Figure 11 C). Fetuin A injected IRI kidneys had higher tissue levels of human Fetuin A, indicative of a higher degree of biomineralization in the ischemic kidneys compared to unoperated control kidneys (Figure 11 C). Exogenous Fetuin A was present in the proximal tubules of mice after intravenous administration (Figure 11 D-F). Without being bound by any theory, it could be hypothesized that Fetuin A may be drawn from the blood to sites of tissue damage, counteracting further tissue destruction and lowering its concentration in blood. In conclusion these experiments show that that intravenous administration of Fetuin A results in an increase of Fetuin A in kidney tissue.

**Time course of human Fetuin A in the serum of mice upon intravenous administration.** After 1 day, the detected amount of human Fetuin A in the serum was reduced compared to 1 minute or 1 hour post injection (Figure 12 A) and after 1 week no human Fetuin A was detectable. Mouse Fetuin A was not detected with the sc-133146-HRP anti-human-Fetuin A antibody as can be derived from the fact that PBS-injected animals gave no signals. Normalized ELISA data using the human-Fetuin-A specific DFTA00 ELISA kit showed the decline of intravenous injected human Fetuin A in mouse serum samples (Figure 12 B, C). Compared to the injection time point (1min), the amount of circulating human Fetuin A was reduced to 80 % after 1 hour, and to 8.5 % after 1 day. After 1 week, human Fetuin A was not detectable anymore, similar to PBS-injected control mice (black circles). In conclusion these experiments show that to maintain the presence of human Fetuin A in circulation, the injection has to be repeated every day.

## METHODS

**Animals.** Breeding, genotyping and all animal experiments were conducted according to the Swiss law for the welfare of animals and were approved by the local authorities (Canton of Bern BE96/11, BE105/14 and BE105/17). All mice, including *Ahsg*$^{tm1Mbl}$ mice and *Clcn5*$^{tm1Gug}$ mice were maintained on a C57BL/6 background and were housed in IVC cages with free access to chow and water and a 12h day/night cycle.

**Induction of hypoxia in pregnant mice.** For timed matings, females in breeding were checked for vaginal plugs every morning and if present the time point was set to gestational day (E) 0.5. *Ahsg*KO mice were obtained from heterozygous breeding pairs, also giving rise to heterozygous and wt littermates that were used as controls. Daily food consumption (weight difference of the food initially provided and the food remaining after 24 h) during pregnancy and the maternal weight gain from E0.5 to E18.5 was recorded. For induction of hypoxia, E13.5 pregnant mice were transferred into a hypoxic glove box (Coy Laboratory Products, Grass Lake, USA). The next day, the oxygen content was gradually lowered to 10% within 6-8 hours with intermittent pauses at 16% and 12.5% to acclimatize the animals to the increasing hypoxic conditions. An electric fan inside the chamber maintained adequate air circulation. The $CO_2$ level was kept low by chelating excess $CO_2$ in soda lime (Sigma, 72073) filled cartridges connected to the air circulation system. Excess humidity was absorbed by silica gel orange granulate (Sigma, 1.01969), changed every day. Mice in the caloric control group received food matching the average amount of food consumed by the mice of the hypoxia group. Pregnant hypoxic or control mice were euthanized on E18.5 and fetuses and placentas were collected, weighed and prepared for further analysis. Fetal kidneys were dissected in PBS using a Leica M80 stereoscope. For primary proximal tubular cells (pPTC) preparation, kidneys of 3-4 weeks normoxic mice were isolated. Assessment of renal functions (GFR and proteinuria) was performed in 9 weeks old fetal hypoxic or normoxic.

**Induction of ischemia and administration for therapeutic treatment experiments.** For experiments in Figure 8 and 9 mice were anesthetized with isoflurane. In deeply anesthetized animals a midline abdominal incision was conducted. Ischemia was induced in the left kidney by clamping the renal vessels for 30 min, the right kidney served as a control (Figure 9). Immediately after surgery, mice received the first treatment of either physiological NaCl solution or bovine Fetuin A monomer (100 µg/g body weight) via intraperitoneal injection. Injections were repeated daily for 4 days. Kidneys were isolated on day 5 and analyzed. NaCl solution: Natrium Chloratum «Bichsel» 0.9% (154 mM); (REF 100 0 090, Bichsel, Interlaken, CH). Fetuin A solution: 0.338 EU/ml LPS, 10 mM $Na_3PO_4$, 8 g/l NaCl (137 mM).

**Induction of ischemia and administration for prophylactic treatment (prevention) experiments.** For experiments in Figure 10 mice were injected intravenously with 900 µg Fetuin A 24 h and 3 h before unilateral ischemia. PBS was injected intravenously in control animals. For ischemia induction, mice were anesthetized with isoflurane. In deeply anesthetized animals a midline abdominal incision was conducted and ischemia was induced in the left kidney by clamping the renal vessels for 20 min. The right kidney served as a unoperated control. Kidneys were collected 24 h post operation. Mice for experiment in Figure 10 had an average body weight of 22 g. PBS solution: 2.67 mM KCI, 1.47 mM $KH_2PO_4$, 137.93 mM NaCl, 8.06 mM $Na_2HPO_4$-7x$H_2O$.

**Transcutaneous assessment of glomerular filtration.** The glomerular filtration rate (GFR) was determined in conscious animals as described in Schreiber, A. et al. Transcutaneous measurement of renal function in conscious mice. Am J Physiol Renal Physiol 303, F783-788 (2012). Briefly, the plasma clearance of FITC-sinistrin (Fresenius-Kabi, LI9830076) is measured across the skin using light-emitting diodes with an emission maximum for FITC at 470 nm and a photodiode detecting the fluorescent light with a maximum sensitivity at 525 nm. The decrease in fluorescence intensity over time is then converted into GFR.

**Proteinuria.** Urine protein content was determined using the Bradford Assay. 3 $\mu$l of urine and 150 $\mu$l of 1x Bradford reagent were mixed, incubated at RT for 5 min and absorbance was measured at 595 nm. (5x Bradford reagent was prepared by dissolving 50 mg Brilliant Blue G-250 (Sigma, B-1131) in 24 ml ethanol and 50 ml 85% phosphoric acid, then adjusting the total volume to 100 ml with ultra-pure water). Urine creatinine content was determined using the Jaffe method. 10 $\mu$l of 1:10 diluted urine was mixed with 100 $\mu$l Creatinine reagent, incubated at RT for 10 min and absorbance was measured at 510 nm. (Creatinine reagent consisted of 10 mM picric acid and 250 mM NaOH, pH 13). Finally, the protein creatinine ratio was calculated for each sample.

**Glomerular count.** 100 $\mu$m Z-stack images of whole-mount E18.5 kidneys stained for nephrin (R&D AF3159) were analysed with the open source image processing software Fiji (imaged, version 2.0.0-rc69/1.52i, https://imagej.net/Fiji). In the TrackMate v3.8.0 plugin, the Downsample LoG detector was set to 80.0 pixel for the estimated blob diameter with a 16-pixel threshold and downsampling factor 2. The number of spots per frame were added to mice, the number of glomeruli per kidney was calculated. A 100 $\mu$m distance between frames was chosen to avoid double counting of identical glomeruli in consecutive images, given an average glomerular diameter of 80 $\mu$m.

**Microarray analysis.** Total RNA from male hypoxic, normoxic and caloric control group E18.5 kidneys was isolated using the RNeasy Mini Kit (Qiagen, 74104). Only high quality RNA (RIN>8, 260/280 ratio>2, 260/230 ratio>1.8) was used for further analysis. 100 ng total RNA samples were processed with the Ambion® WT Expression Kit (4411973, life technologies) kit. 5.5 $\mu$g of the cDNA was fragmented and labelled with GeneChip® WT Terminal Labeling kit (901525, Affymetrix). 2.3 $\mu$g biotinylated fragments were hybridized to Affymetrix Mouse Gene 1.0 ST arrays at 45 °C for 16 h, washed and stained according to the protocol described in Affymetrix GeneChip® Expression Analysis Manual (Fluidics protocol FS450_0007). The arrays were scanned with Affymetrix GeneChip® Scanner 3000 7G and raw data was extracted from the scanned images and analysed with the Affymetrix Power Tools software package. Hybridization quality was assessed using Affymetrix Expression Console software (version 1.1.2800.28061). Normalized expression signals were calculated from Affymetrix CEL files by the Robust Multi-array Average algorithm (RMA). Differential hybridized features were identified using the R Bioconductor package "limma" that implements linear models for microarray data. P values were adjusted for multiple testing with Benjamini and Hochberg's method to control the false discovery rate (FDR). Probe sets showing at least 1.3-fold change and a FDR<0.05 were considered significant. Differential expression values between hypoxic, normoxic and caloric control group E18.5 kidneys were mapped with Heatmapper (http://www.heatmapper.ca) using average linkage and Euclidean distance measurement. For functional annotation, GO-term analysis was performed using the DAVID platform (https://david.ncifcrf.gov).

**RT-qPCR.** Total RNA was isolated using TRIzol® reagent (Invitrogen 15596026) according to the manufacturer's protocol. RNA concentration and quality was determined with a Nanodrop 1000 spectrophotometer (ThermoFisher Scientific, Switzerland) and 1000 ng were transcribed into cDNA using PrimeScript RT Reagent Kit (Takara, RR037A). cDNA was diluted to 2 ng/$\mu$l and qPCR was performed with either TaqMan Gene Expression Assays (ThermoFisher) or FAM-labelled UPL probe (Roche) plus corresponding gene-specific primers and TaqMan Fast Universal PCR Master mix (Applied Biosystems, 4352042) on a 7500 Fast Real-Time PCR System (Applied Biosystems). Data analysis was performed with Microsoft Excel. The $2^{(-\Delta Ct)}$ method was used to calculate the relative expression levels for RT-qPCR.

**Identification of HIF binding sites.** For the identification of putative HIF binding sites, the 20 kb genomic region overlapping with the *Ahsg* locus (10 kb up- and downstream of the start codon) of 15 different species (cat, chicken, chimp, cow, dog, ghost shark, horse, human, mouse, pig, rabbit, rat, sheep, xenopus, zebrafish) was analysed with the JASPAR database (http://jaspar.genereg.net). The relative profile score threshold was set to 90%. For enrichment analysis, putative sites of all species were clustered in 1kb windows. HREs with a relative score >0.9, >0.93 and >0.97 are shown. For the alignment of human HIF alpha sites identified by ChIP-seq of hypoxic MCF7 cells (cf. Schödel J, Oikonomopoulos S, Ragoussis J, Pugh CW et al. Blood 2011 Jun 9;117(23):e207-17.; Series GSE28352; https://www.ncbi.nlm.nih.gov/ geo/query/acc.cgi?acc=GSE28352) with active regulatory marks of the AHSG locus, the HIF-1-alpha and HIF-2-alpha data sets encompassing chr3: 180,000,000-190,000,000 (including the AHSG locus) were converted into BAM files using the web-based Galaxy platform (https://usegalaxy.org) and uploaded to the human assembly GRCh37/hg19 on the USCS genome browser (https://genome.ucsc.edu). To this alignment, the following data sets were added: layered chromatin marks often found near active regulatory elements of 7 cell lines (H3K27Ac and H3K4Me1, ENCODE) and open chromatin of hypoxic MCF7 cells (DNasel HS, ENCODE). For the mouse Ahsg locus, the potential HIF binding sites identified with JASPAR were aligned with data sets (DNaseI HS, ENCODE/UW; H3K27Ac and H3K4Me1, ENCODE/LICR) derived from 8 weeks mouse liver, heart and kidney, showing the mean signal intensity (bar graphs, auto-scaled, log-transformed, smoothened (16 pixels)).

**Molecular cloning.** For luciferase assays, the 2.5 kb promoter fragment upstream of the mouse Ahsg ATG was amplified from genomic DNA (C57BL/6) using specific primers and PrimeSTAR® GXL DNA Polymerase (Takara, R050A). The 500 bp promoter fragments (wt and mutant) and the 500 bp fragment of intronic sequences (wt and mutant) were synthesized by IDTDNA (https://eu.idtdna.com/pages). The promoter fragments were inserted into the pGL3-basic-P2P-607 plasmid with NcoI and SacI restriction enzymes (both NEB, R3193S and R3156S). Intronic fragments were inserted into the pGL3- basic vector or the pGL3-basic vector carrying the promoter fragments using BamHI and SalI restriction enzymes (both NEB, R3136S and R3138S). For in-situ hybridization, the cDNA of exons 2-5 of mouse Ahsg was obtained from IDTDNA and cloned into pBluescriptII KS- using SpeI and EcoRI restriction enzymes (both NEB, R3133S and R3101S).

**Luciferase assay.** 24 h after transfection, cells were washed twice with PBS, lysed (250 mM KCl, 50 mM Tris/$H_3PO_4$ pH 7.8, 10% glycerol, 0.1% NP40) on ice for 20 min and centrifuged at 14000 rpm at 4 °C for 10 min. Of the supernatant 10 $\mu$l were used for each reaction. Injection of reaction solutions (Luciferase: 100 $\mu$l of 25 mM Tris/$H_3PO_4$ pH 7.8, 10 mM MgSO4, 2 mM ATP pH 7.5, 50 $\mu$M luciferin; Renilla: 100 $\mu$l of 50 mM Tris/HCl pH 7.6, 100 mM NaCl, 1 mM EDTA, 0.5 $\mu$M coelenterazine) and activity measurement was performed with a Fluoroskan Ascent FL (ThermoFisher). Each sample was measured in duplicates and luciferase activity was normalized by renilla activity.

**Whole-mount in situ hybridization.** E18.5 kidneys were fixed in 4 % PFA, dehydrated and stored in methanol at -20 °C. In situ hybridization using a digoxigenin-labelled riboprobe was performed as described in Rudloff, S. & Kemler, R. Differential requirements for beta-catenin during mouse development. Development 139, 3711-3721 (2012). Probes were generated using the DIG RNA Labeling Mix (Roche, 11175025910) and T3 or T7 RNA polymerase (both Roche, 11031163001 or 10881767001). An alkaline phosphatase-conjugated antibody was used to detect the DIG-labelled probes (Roche, 11093274910).

**TUNEL staining.** Fragmented DNA in apoptotic cells was detected using the Promega DeadEnd Colorimetric TUNEL System (G7360) according to the manufacturer.

**Histochemistry.** For immunohistochemistry, PFA-fixed, paraffin-embedded tissue sections were rehydrated and endogenous peroxidase was blocked by incubating the slides in 1.5 % $H_2O_2$ solution (0.02 M citric acid, 0.06 M $Na_2HPO_4$) at RT for 15 min in the dark. Antigen retrieval was performed by boiling in Tris-EDTA buffer pH 9 for 20 min followed by slow cool down to RT. After blocking in 2 % BSA in PBS at RT for 1 h, the sections were incubated with primary antibodies in blocking solution o/n at 4 °C. Following three washing steps in PBS, the sections were incubated with HRP-conjugated secondary antibodies (mouse or rabbit: Dako EnVision+ System from Agilent (K4001 or K4003), goat: SCBT, sc-2304) for 1 h at RT. After three washing steps in PBS, the signal was developed with DAB (Agilent, K3468). The sections were counterstained with Harris haematoxylin solution (Sigma, HHS16), dehydrated and mounted using Eukitt medium (Sigma, 03989). For Picrosirius red staining of collagen, dewaxed, rehydrated tissue sections were incubated in staining solution (0.5 g Direct Red 80 in saturated aqueous solution of picric acid (both Sigma, 365548 and P6744)) for 1 h at RT. After washing twice in acidified water (0.5% glacial acetic acid), the sections were dehydrated and mounted using Eukitt.

**Immunofluorescent staining.** Cryosections were fixed in 4 % PFA at RT for 10 min, washed twice in PBS and permeabilised by incubation in PBST (0.1% Triton X-100 in PBS) at RT for 10 min. After blocking in 10 % FCS, 0.5 % Tween-20 in PBS at RT for 1 h, the sections were incubated with primary antibodies in blocking solution o/n at 4 °C. Following three washing steps in PBS, the sections were incubated with fluorescence-conjugated secondary antibodies in blocking solution in the dark for 1 h at RT. DNA was stained with DAPI 1:5000 in PBS. Sections were mounted in MOWIOL solution (2.4 g MOWIOL 4-88 reagent (Merck, 475904) in 6g glycerol and 18 ml 0.13 M Tris pH 8.5). For whole mount immunofluorescence staining of E18.5 kidneys, the iDisco staining protocol (https://idisco.info/idisco-protocol/) with methanol pre-treatment was applied. An incubation time n=1 day and a solution volume of 1.6 ml was used for the relevant steps. The kidneys were mounted in 8-well glass chamber slides (ThermoFisher, 154534) and imaged immediately.

**Fluorescent detection of calcium.** Thick cryosections (30 $\mu$m) were incubated with 10 ng/ml ATTO 488 fluorescently labelled Fetuin A (in calcium-free PBS) in the dark at RT for 60 min, rinsed 3 times with PBS and mounted with MOWIOL solution. Nuclei were counterstained with DAPI.

**Imaging.** Fluorescence imaging was performed on a IMIC digital microscope (FEI, Type 4001) using the Polychrome V light source, an Orca-$R^2$ camera controller from Hamamatsu (C10600) and Live Acquisition software (FEI, version 2.6.0.14). Image analysis was performed using Offline Analysis software (FEI). Bright field imaging was performed on a Nikon E600 microscope equipped with Nikon objectives (Plan Fluor ELWD 20x/0.45, Plan Apo 40x/1.0 Oil and 60x/1.40 Oil) using a Digital Sight DS-UE camera controller and DSRi1 camera (both Nikon). Image analysis was performed using Nikon software NIS Elements 4.0.

**ELISA.** Mouse Fetuin A levels, e.g. in serum or renal tissue, were determined using the mouse-specific Fetuin A/AHSG Quantikine ELISA Kit (R&D, MFTA00) according to the manufacturer bio-techne. Human Fetuin A levels, e.g. in serum or renal tissue, were determined using the human-specific Fetuin A/AHSG Quantikine ELISA Kit (R&D, DFTA00) according to the manufacturer bio-techne.

**Cell culture.** The normal rat kidney (NRK) cell line was cultured in DMEM (Gibco, 41965- 039) and 10% fetal bovine serum (FBS). The human kidney (HK-2) cell line was cultured in Keratinocyte-SFM medium (Gibco, 17005-075). For luciferase assays, NRK cells were transfected with luciferase reporter plasmids and pCMV-Renilla (10% of total transfected DNA, used for normalization) using jetPrime® reagent (Polyplus, 114-07), stimulated with 1 mM DMOG (Echelon Biosciences, F-0010) 6 h after and harvested 24 h after transfection. For hypoxia, cell culture was performed at 0.2 % oxygen for 48 h. Primary proximal tubular cells (pPTC) were isolated from 3-4 weeks old kidneys (cf. Terryn, S. et al. A primary culture of mouse proximal tubular cells, established on collagen-coated membranes. Am J Physiol Renal Physiol 293, F476-485 (2007)). Briefly, proximal tubular fragments were obtained by digesting cortical kidney tissue with collagenase and filtration through an 80 μm pore size membrane. pPTCs were cultured in DMEM/F12 (Gibco, 21041-025) supplemented with 15 mM HEPES, 0.55 mM NaPyruvate, 1% NEAA and renal epithelial cell growth medium (REGM) supplements (Lonza, CC-4127). Instead of FBS, serum from Ahsg KO mice was used. Upon confluency, pPTCs were split and replated once using Accutase solution (Sigma, A6964). For 24 hypoxia, cell culture was performed at 0.2 % oxygen for 48 h. 100 μg/ml Fetuin A (Sigma, F3385) or bovine serum albumin (BSA, Sigma, A3059) was added to the culture medium 48 h before the end of the experiment. Before treatment with 5 ng/ml rmTGF-β1 (R&D, 7666-MB) for 5 min, cells were starved for 24 h.

**Western blot analysis.** Total protein lysates were obtained using RIPA buffer (Sigma, R0278) supplemented with protease inhibitors (Roche, 11836153001). Proteins were separated by SDS-PAGE and blotted onto PVDF membranes (ThermoFisher, 88518). Upon blocking with 5 % milk in TBST, the membranes were incubated with primary antibodies at 4 °C o/n, incubation with HRP-conjugated secondary antibodies was performed at RT for 1 h. The signal was detected with ECL (GE Healthcare, RPN2106) or SuperSignal (ThermoFisher, 34076) depending on signal intensity. Densiometric analysis was performed with the open source image processing software Fiji (imaged, version 2.0.0-rc69/1.52i, https://imagej.net/Fiji).

**Calculation of concentration for intraperitoneal injected bovine Fetuin A in the mouse.** The reference blood volume of mice was 58.5 ml/kg (nc3rs.org.uk), 77-80 ml/kg (jax.org). Based on these values, 68.5 ml/kg was used for calculations. The calculated molecular weight of bovine Fetuin A (UniProtKB - P12763 (FETUA_BOVIN)) is 38.4 kDa. Thus, a concentration of 38 μM in the blood would be reached, if all injected Fetuin A was in the blood or if intravenous injection of Fetuin A was performed. Furthermore, Fetuin A undergoes posttranslational modifications. Here, especially glycosylation plays a predominant role, increasing the molecular weight to 51-67 kDa. Therefore, a molecular weight of 60 kDa was used in the calculation (where 1 Da = 1 g/mol). This results in a concentration of 24.3 μM in the blood of injected mice, if all injected Fetuin A was in the blood or if intravenous injection of Fetuin A was performed and based on the injected amount of 100 μg/g body weight:

$$100\,\frac{\text{mg}}{\text{kg}} \times \left(68.5\,\frac{\text{ml}}{\text{kg}}\right)^{-1} \times (60\,\text{kDa})^{-1} = 24.3\,\frac{\mu\text{mol}}{\text{L}}$$

However, intraperitoneal injections were performed, which results in lower concentrations in the blood, because Fetuin A has to be transported from the abdominal cavity into circulation. During this process, not all molecules will be absorbed, due to partial degradation in the cavity or pinocytosis and metabolization by mesothelial cells. In a study (Regoeczi et al (1989), Am J Physiol.; 256(4 Pt 1): E447-52), it was shown that intraperitoneal injection of albumin reached approximately 40 % of the dose in the blood after 2-3 hours compared to the intravenous injection of the same amount of albumin. Based on a 40 % uptake in mouse the resulting concentration after intraperitoneal injection is:

$$100\,\frac{\text{mg}}{\text{kg}} \times \left(68.5\,\frac{\text{ml}}{\text{kg}}\right)^{-1} \times (60\,\text{kDa})^{-1} \times 0.4 = 9.7\,\frac{\mu\text{mol}}{\text{L}}$$

Therefore, if 100 mg/kg body weight of Fetuin A is injected intraperitoneal, the resulting concentration in blood would be about 9.7 μM. As described above, it was found that such a relatively low dose is sufficient to treat renal ischemic damages.

**Calculation of concentration for intravenous injected human Fetuin A in the mouse.** The reference blood volume of mice was 58.5 ml/kg (nc3rs.org.uk), 77-80 ml/kg (jax.org). Based on these values, 68.5 ml/kg was used for calculations. The calculated molecular weight of human Fetuin A (UniProtKB - P12763 (FETUA_BOVIN)) is 38.4 kDa. Furthermore, Fetuin A undergoes posttranslational modifications. Here, especially glycosylation plays a predominant role, increasing the molecular weight to 51-67 kDa. Therefore, a molecular weight of 60 kDa was used in the calculation.

In the experiments for prophylactic treatment, the mice had an average body weight of 22 g. For a mouse with a body weight of 22 g the corresponding blood volume is about

$$\frac{68.5\,\text{mL}}{1000\,\text{g}} \times 22\,\text{g} = 1.51\,\text{ml}$$

With an injection of 900 μg of human Fetuin A this results in a concentration in blood of

$$\frac{900\,\mu\text{g}}{1.51\,\text{mL}} \times (60\,\text{kDa})^{-1} = 9.9\,\frac{\mu\text{mol}}{\text{L}}$$

Therefore, if 900 μg of human Fetuin A is injected intravenously in mice having an average body weight of 22 g, the resulting concentration in blood would be about 9.9 μM. As described above, it was found that such a relatively low dose is sufficient to prevent renal ischemic damages.

**Calculation of concentration and dosing for human Fetuin A in humans.** The reference blood volume for a human adult is 77 ml/kg (male) and 65 ml/kg (female), which is an average volume of 71 ml/kg. The molecular weight of human Fetuin A (UniProtKB - P02765 (FETUA_HUMAN) is generally comparable to bovine Fetuin A, i.e. also about 60 kDa (with glycosylation). For administration of 10 mg/kg (iv), 25 mg/kg (iv), 50 mg/kg (iv) of Fetuin A to a human adult the resulting concentration in human blood would be:

$$10\,\frac{\text{mg}}{\text{kg}} \times \left(71\,\frac{\text{ml}}{\text{kg}}\right)^{-1} \times (60\,\text{kDa})^{-1} = 2.35\,\mu\text{M}$$

$$25\,\frac{\text{mg}}{\text{kg}} \times \left(71\,\frac{\text{ml}}{\text{kg}}\right)^{-1} \times (60\,\text{kDa})^{-1} = 5.87\,\mu\text{M}$$

$$50\,\frac{\text{mg}}{\text{kg}} \times \left(71\,\frac{\text{ml}}{\text{kg}}\right)^{-1} \times (60\,\text{kDa})^{-1} = 11.74\,\mu\text{M}$$

Similarly, it can be calculated which dose corresponds to the above mentioned concentration of 9.7 μM:

$$9.7\,\mu\text{M} \times 71\,\frac{\text{ml}}{\text{kg}} \times 60\,\text{kDa} = 41.3\,\frac{\text{mg}}{\text{kg}}$$

Similarly, it can be calculated which dose corresponds to the above mentioned concentration of 9.9 μM:

$$9.9\,\mu\text{M} \times 71\,\frac{\text{ml}}{\text{kg}} \times 60\,\text{kDa} = 42.2\,\frac{\text{mg}}{\text{kg}}$$

Therefore, the calculated equivalent to 100 μg/g body weight of Fetuin A being injected intraperitoneal to a mouse, is about 41.3 mg/kg when injected intravenously into an adult human and the calculated equivalent to 900 μg of Fetuin A being injected intravenously to a mouse with a body weight of 22 g is about 42.2 mg/kg when injected intravenously into an adult human.

**Data analysis.** Statistical analysis and graphs were performed with Prism 7 (https://www.graphpad.com). Two groups were compared by t-tests, multiple groups by one-way ANOVA (Tukey's multiple comparisons test, unless specified otherwise). ****, P<0.0001; ***, P<0.001; **, P<0.01; *, P<0.05; ns, not significant; Error bars, standard deviation (SD); Whiskers, min to max.

**Claims**

1. Fetuin A (AHSG) for use in a method for treating a renal disorder, wherein an amount of Fetuin A effective for treating the renal disorder is administered to a subject in need thereof, wherein the renal disorder is an ischemic renal disorder.

2. Fetuin A for use in a method according to claim 1, wherein the ischemic renal disorder is selected from the group consisting of ischemic acute renal disorders, ischemic chronic renal disorders, kidney fibrosis resulting from ischemia, renal inflammation resulting from ischemia, acute kidney injuries resulting from ischemia, disorders related to kidney hypoxia, renal ischemia-reperfusion injury, ischemic kidney tissue damage, disorders related to ischemia during kidney transplantation and combinations thereof..

3. Fetuin A for use in a method according to claim 2, wherein the renal disorder is selected from the group consisting of delayed graft function, organ rejection of kidney transplants, kidney tissue damage resulting from kidney transplantation, inflammation resulting from kidney transplantation, ischemia-reperfusion injury resulting from kidney transplantation, and combinations thereof.

4. Fetuin A for use in a method according to any one of the preceding claims, wherein the renal disorder is caused by hypoxia during surgery, preferably wherein the hypoxia is caused by ischemia during kidney transplantation or during cardiovascular surgery.

5. Fetuin A for use in a method according to any one of the preceding claims, wherein the Fetuin A is human Fetuin A, preferably wherein the Fetuin A is derived from human blood plasma.

6. Fetuin A for use in a method according to any one of the preceding claims, wherein the Fetuin A is at least 90%, at least 95% or at least 99% identical and/or homologous to SEQ ID NO: 1.

7. Fetuin A for use in a method according to any one of the preceding claims, wherein the Fetuin A comprises or consists of SEQ ID NO: 1 or fragments thereof.

8. Fetuin A for use in a method according to any one of the preceding claims, wherein the Fetuin A is recombinantly expressed, preferably in a host cell system providing posttranslational modifications, more preferably glycosylation.

9. Fetuin A for use in a method according to any one of the preceding claims, wherein the Fetuin A is administered to the subject by way of injection or inhalation.

10. Fetuin A for use in a method according to claim 9, wherein the Fetuin A is injected intravenously to the subject.

11. Fetuin A for use in a method according to any one of the preceding claims, wherein the Fetuin A is administered to the subject in a concentration of from 1 to 200 mg/kg (of body weight), from 5 to 100 mg/kg (of body weight) or from 10 to 50 mg/kg (of body weight).

12. Fetuin A for use in a method according to any one of the preceding claims, wherein the treatment comprises removal of calcium mineral depositions in transplanted kidney tissue.

13. Fetuin A for use in a method according to any one of the preceding claims, wherein at least two or at least three doses of the Fetuin A are administered.

14. Fetuin A for use in a method according to any one of the preceding claims, wherein at least one dose of Fetuin A is administered prior, during or after an ischemic incident and/or surgery, in particular wherein the ischemic incident and/or the surgery is a kidney transplantation or cardiovascular surgery.

15. Fetuin A for use in a method according to claim 14, wherein at least one dose of Fetuin A is administered within 48 hours before an ischemic incident and/or surgery or wherein at least one dose of Fetuin A is administered within 48 hours after an ischemic incident and/or surgery.

16. Fetuin A for use in a method according to any one of the preceding claims, wherein Fetuin A is administered in divided doses over a total duration of 1 to 50 days, in particular over a total duration of 1 to 25 days or over a total duration of 2 to 10 days.

17. Pharmaceutical composition for use in treating an renal disorder, comprising Fetuin A and optionally at least one pharmaceutical acceptable carrier, wherein the renal disorder is an ischemic renal disorder.

18. The pharmaceutical composition for use in treating a renal disorder of claim 17, wherein the renal disorder is caused by hypoxia during surgery, preferably wherein the hypoxia is caused by ischemia during kidney transplantation or during cardiovascular surgery.

19. The pharmaceutical composition for use in treating a renal disorder according to claim 17 or 18, wherein the Fetuin A is defined as in any one of claims 5 to 16.


**Patentansprüche**

1. Fetuin A (AHSG) zur Verwendung in einem Verfahren zur Behandlung einer Nierenerkrankung, wobei eine zur Behandlung der Nierenerkrankung wirksame Menge an Fetuin A einem Individuum verabreicht wird, das dies benötigt, wobei die Nierenerkrankung eine ischämische Nierenerkrankung ist.

2. Fetuin A zur Verwendung in einem Verfahren nach Anspruch 1, wobei die ischämische Nierenerkrankung ausgewählt ist aus der Gruppe bestehend aus ischämischen akuten Nierenerkrankungen, ischämischen chronischen Nierenerkrankungen, Nierenfibrose infolge von Ischämie, Nierenentzündung infolge von Ischämie, akuten Nierenschädigungen infolge von Ischämie, Störungen im Zusammenhang mit Nierenhypoxie, renalen Ischämie-Reperfusionsschäden, ischämischen Nierengewebeschäden, Störungen im Zusammenhang mit Ischämie während einer Nierentransplantation und Kombinationen davon.

3. Fetuin A zur Verwendung in einem Verfahren nach Anspruch 2, wobei die Nierenerkrankung ausgewählt ist aus der Gruppe bestehend aus verzögerter Transplantatfunktion, Organabstoßung von Nierentransplantaten, Schädigung des Nierengewebes infolge einer Nierentransplantation, Entzündung infolge einer Nierentransplantation, Ischämie-Reperfusionsschäden infolge einer Nierentransplantation und Kombinationen davon.

4. Fetuin A zur Verwendung in einem Verfahren nach einem der vorhergehenden Ansprüche, wobei die Nierenerkrankung durch Hypoxie während einer chirurgischen Operation verursacht wird, wobei die Hypoxie vorzugsweise durch Ischämie während einer Nierentransplantation oder während einer kardiovaskulären Operation verursacht wird.

5. Fetuin A zur Verwendung in einem Verfahren nach einem der vorhergehenden Ansprüche, wobei das Fetuin A menschliches Fetuin A ist, wobei das Fetuin A vorzugsweise aus menschlichem Blutplasma abgeleitet ist.

6. Fetuin A zur Verwendung in einem Verfahren nach einem der vorhergehenden Ansprüche, wobei das Fetuin A zu mindestens 90 %, mindestens 95 % oder mindestens 99 % identisch und/oder homolog zu SEQ ID NO: 1 ist.

7. Fetuin A zur Verwendung in einem Verfahren nach einem der vorhergehenden Ansprüche, wobei das Fetuin A SEQ ID NO: 1 oder Fragmente davon umfasst oder daraus besteht.

8. Fetuin A zur Verwendung in einem Verfahren nach einem der vorhergehenden Ansprüche, wobei das Fetuin A rekombinant exprimiert wird, vorzugsweise in einem Wirtszellsystem, das posttranslationale Modifikationen, stärker bevorzugt Glykosylierung, bereitstellt.

9. Fetuin A zur Verwendung in einem Verfahren nach einem der vorhergehenden Ansprüche, wobei das Fetuin A dem Individuum durch Injektion oder Inhalation verabreicht wird.

10. Fetuin A zur Verwendung in einem Verfahren nach Anspruch 9, wobei das Fetuin A dem Individuum intravenös injiziert wird.

11. Fetuin A zur Verwendung in einem Verfahren nach einem der vorhergehenden Ansprüche, wobei das Fetuin A dem Individuum in einer Konzentration von 1 bis 200 mg/kg (Körpergewicht), 5 bis 100 mg/kg (Körpergewicht) oder 10 bis 50 mg/kg (Körpergewicht) verabreicht wird.

12. Fetuin A zur Verwendung in einem Verfahren nach einem der vorhergehenden Ansprüche, wobei die Behandlung die Entfernung von Calcium-Mineralablagerungen in transplantiertem Nierengewebe umfasst.

**13.** Fetuin A zur Verwendung in einem Verfahren nach einem der vorhergehenden Ansprüche, wobei mindestens zwei oder mindestens drei Dosen des Fetuin A verabreicht werden.

**14.** Fetuin A zur Verwendung in einem Verfahren nach einem der vorhergehenden Ansprüche, wobei mindestens eine Dosis Fetuin A vor, während oder nach einem ischämischen Vorfall und/oder einer Operation verabreicht wird, insbesondere wobei es sich bei dem ischämischen Vorfall und/oder der Operation um eine Nierentransplantation oder eine kardiovaskuläre Operation handelt.

**15.** Fetuin A zur Verwendung in einem Verfahren nach Anspruch 14, wobei mindestens eine Dosis Fetuin A innerhalb von 48 Stunden vor einem ischämischen Vorfall und/oder einer Operation verabreicht wird oder wobei mindestens eine Dosis Fetuin A innerhalb von 48 Stunden nach einem ischämischen Vorfall und/oder einer Operation verabreicht wird.

**16.** Fetuin A zur Verwendung in einem Verfahren nach einem der vorhergehenden Ansprüche, wobei Fetuin A in aufgeteilten Dosen über eine Gesamtdauer von 1 bis 50 Tagen, insbesondere über eine Gesamtdauer von 1 bis 25 Tagen oder über eine Gesamtdauer von 2 bis 10 Tagen verabreicht wird.

**17.** Pharmazeutische Zusammensetzung zur Verwendung bei der Behandlung einer Nierenerkrankung, umfassend Fetuin A und optional mindestens einen pharmazeutisch akzeptablen Träger, wobei die Nierenerkrankung eine ischämische Nierenerkrankung ist.

**18.** Pharmazeutische Zusammensetzung zur Verwendung bei der Behandlung einer Nierenerkrankung nach Anspruch 17, wobei die Nierenerkrankung durch Hypoxie während einer Operation verursacht wird, wobei die Hypoxie vorzugsweise durch Ischämie während einer Nierentransplantation oder während einer kardiovaskulären Operation verursacht wird.

**19.** Pharmazeutische Zusammensetzung zur Verwendung bei der Behandlung einer Nierenerkrankung nach Anspruch 17 oder 18, wobei das Fetuin A wie in einem der Ansprüche 5 bis 16 definiert ist.

## Revendications

**1.** Fétuine A (AHSG) pour une utilisation dans un procédé pour le traitement d'un trouble rénal, une quantité de fétuine A efficace pour traiter le trouble rénal étant administrée à un sujet qui en a besoin, le trouble rénal étant un trouble rénal ischémique.

**2.** Fétuine A pour une utilisation dans un procédé selon la revendication 1, le trouble rénal ischémique étant choisi dans le groupe constitué par des troubles rénaux aigus ischémiques, des troubles rénaux chroniques ischémiques, la fibrose rénale résultant de l'ischémie, une inflammation rénale résultant de l'ischémie, des lésions rénales aiguës résultant de l'ischémie, des troubles liés à l'hypoxie rénale, une lésion d'ischémie-reperfusion rénale, une lésion de tissus rénaux ischémiques, des troubles liés à l'ischémie pendant une transplantation rénale et des combinaisons correspondantes.

**3.** Fétuine A pour une utilisation dans un procédé selon la revendication 2, le trouble rénal étant choisi dans le groupe constitué par une fonction de greffe retardée, un rejet d'organe de transplantation rénale, une lésion de tissus rénaux résultant d'une transplantation rénale, une inflammation résultant d'une transplantation rénale, une lésion d'ischémie-reperfusion résultant d'une transplantation rénale et des combinaisons correspondantes.

**4.** Fétuine A pour une utilisation dans un procédé selon l'une quelconque des revendications précédentes, le trouble rénal étant causé par l'hypoxie pendant une chirurgie, préférablement l'hypoxie étant causée par une ischémie pendant une transplantation rénale ou pendant une chirurgie cardiovasculaire.

**5.** Fétuine A pour une utilisation dans un procédé selon l'une quelconque des revendications précédentes, la fétuine A étant la fétuine A humaine, préférablement la fétuine A étant issue de plasma sanguin humain.

**6.** Fétuine A pour une utilisation dans un procédé selon l'une quelconque des revendications précédentes, la fétuine A étant au moins 90 %, au moins 95 % ou au moins 99 % identique et/ou homologue à la SEQ ID NO: 1.

**7.** Fétuine A pour une utilisation dans un procédé selon l'une quelconque des revendications précédentes, la fétuine A comprenant ou étant constituée de la SEQ ID NO: 1 ou de fragments correspondants.

**8.** Fétuine A pour une utilisation dans un procédé selon l'une quelconque des revendications précédentes, la fétuine A étant exprimée de manière recombinante, préférablement dans un système de cellule hôte fournissant des modifications posttranslationnelles, plus préférablement une glycosylation.

**9.** Fétuine A pour une utilisation dans un procédé selon l'une quelconque des revendications précédentes, la fétuine A étant administrée au sujet au moyen d'une injection ou d'une inhalation.

**10.** Fétuine A pour une utilisation dans un procédé selon la revendication 9, la fétuine A étant injectée de manière intraveineuse au sujet.

**11.** Fétuine A pour une utilisation dans un procédé selon l'une quelconque des revendications précédentes, la fétuine A étant administrée au sujet en une concentration allant de 1 à 200 mg/kg (de poids corporel), de 5 à 100 mg/kg (de poids corporel) ou de 10 à 50 mg/kg (de poids corporel).

**12.** Fétuine A pour une utilisation dans un procédé selon l'une quelconque des revendications précédentes, le traitement comprenant l'élimination de dépôts minéraux de calcium dans un tissu rénal transplanté.

**13.** Fétuine A pour une utilisation dans un procédé selon l'une quelconque des revendications précédentes, au moins deux ou au moins trois doses de la fétuine A étant administrées.

**14.** Fétuine A pour une utilisation dans un procédé selon l'une quelconque des revendications précédentes, au moins une dose de fétuine A étant administrée avant, pendant ou après un incident ischémique et/ou une chirurgie, en particulier l'incident ischémique et/ou la chirurgie étant une transplantation rénale ou une chirurgie cardiovasculaire.

**15.** Fétuine A pour une utilisation dans un procédé selon la revendication 14, au moins une dose de fétuine A étant administrée dans les 48 heures avant un incident ischémique et/ou une chirurgie ou au moins une dose de fétuine A étant administrée dans les 48 heures après un incident ischémique et/ou une chirurgie.

**16.** Fétuine A pour une utilisation dans un procédé selon l'une quelconque des revendications précédentes, la fétuine A étant administrée en doses divisées sur une durée totale de 1 à 50 jours, en particulier sur une durée totale de 1 à 25 jours ou sur une durée totale de 2 à 10 jours.

**17.** Composition pharmaceutique pour une utilisation dans le traitement d'un trouble rénal, comprenant de la fétuine A et éventuellement au moins un support pharmaceutiquement acceptable, le trouble rénal étant un trouble rénal ischémique.

**18.** Composition pharmaceutique pour une utilisation dans le traitement d'un trouble rénal selon la revendication 17, le trouble rénal étant causé par une hypoxie pendant une chirurgie, préférablement l'hypoxie étant causée par une ischémie pendant une transplantation rénale ou pendant une chirurgie cardiovasculaire.

**19.** Composition pharmaceutique pour une utilisation dans le traitement d'un trouble rénal selon la revendication 17 ou 18, la fétuine A étant définie comme dans l'une quelconque des revendications 5 à 16.

FIGURE 1 A

FIGURE 1 B

## Litter size

**FIGURE 1 C**

**Fetal mass**

**FIGURE 1 D**

**Nephron number
(Glomeruli)**

**FIGURE 2 A**

**FIGURE 2 B**

*Ahsg*

**FIGURE 2 C**

*Apoa2*

**FIGURE 2 D**

*Fgg*

**FIGURE 2 E**

*Gys2*

**FIGURE 2 F**

Fetuin-A ELISA

**FIGURE 3 A-L**

**FIGURE 4 A**

**FIGURE 4 B**

**Luciferase activity**

**FIGURE 4 C**

**FIGURE 4 D**

**FIGURE 4 E**

**FIGURE 5 A**

Proteinuria

**FIGURE 5 B**

Glomerular filtration rate

**FIGURE 5 C-D**

**FIGURE 5 E**

**FIGURE 5 F**

*Col1a1*

**FIGURE 5 G**

*Fn1*

**FIGURE 6 A-I**

**FIGURE 6 J-M**

**FIGURE 6 N**

F4/80

**FIGURE 7 A**

**FIGURE 7 B**

*Acta2*

**FIGURE 7 C**

*Col1a1*

**FIGURE 7 D**

*Fn1*

**FIGURE 7 E**

*Acta2*

**FIGURE 7 F**

*Col1a1*

**FIGURE 7 G**

**FIGURE 7 H**

**FIGURE 7 I**

**FIGURE 8**

**FIGURE 9 A**

*Col1a1*

**FIGURE 9 B**

Col3a1

**FIGURE 9 C**

Col6a1

**FIGURE 9 D**

*Acta2*

**FIGURE 9 E**

*Fn1*

**FIGURE 9 F**

*Vim*

**FIGURE 10 A**

*Kim1*

**Figure 10 B**

*Krt18*

**Figure 10 C**

*Krt20*

**Figure 10 D**

*Lcn2*

**Figure 10 E**

*Clu*

**Figure 10 F**

*Tgfb1*

**Figure 10 G**

*Vim*

**Figure 10 H**

*Col1a1*

**Figure 10 I**

*Col3a1*

**Figure 10 J**

*Col6a1*

**Figure 10 K**

*Arg1*

**Figure 10 L**

***Ccl2***

**Figure 10 M**

***Il6***

**Figure 10 N**

*Tnf*

**Figure 11A**

**Figure 11 B**

Mouse Fetuin-A
(renal tissue)

**Figure 11 C**

Human Fetuin-A
(renal tissue)

**Figure 11 D**

15 min post injection
Alexa-488-labeled Fetuin-A

**Figure 11 E**

30 min post injection
Alexa-488-labeled Fetuin-A

**Figure 11 F**

60 min post injection
Alexa-488-labeled Fetuin-A
DAPI

G

PT

PT

**Figure 12 A**

A B C D E F G H I J K

50 kDa

human
Fetuin-A

1min 1hour 1day 1week PBS

after i.v. injection

**Figure 12 B**

Kinetics of human Fetuin-A
(in mouse serum)

**Figure 12 C**

Kinetics of human Fetuin-A
(in mouse serum)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0060943 A
- WO 2005007199 A

**Non-patent literature cited in the description**

- **WANG.** *J Cereb Blood Flow Metab,* 2010, vol. 30 (3), 493-504
- **WEICKERT.** *Circulation,* 2008, vol. 118, 2555-2562
- **ROOS.** *Kidney Blood Press Res.,* 2011, vol. 34 (5), 328-33
- **MERSAI.** *Urol J,* 2015, vol. 12 (3), 2182-6
- **KOCA.** *Nephrology Dialysis Transplantation,* 2017, vol. 32 (3), iii419-iii420
- **ALTSCHUL et al.** *J. Mol. Biol;,* 1990, vol. 215 (3), 403-410
- **SCHREIBER, A. et al.** Transcutaneous measurement of renal function in conscious mice. *Am J Physiol Renal Physiol,* 2012, vol. 303, F783-788
- **SCHÖDEL J ; OIKONOMOPOULOS S ; RAGOUSSIS J ; PUGH CW et al.** *Blood,* 09 June 2011, vol. 117 (23), e207-17, https://www.ncbi.nlm.nih.gov/geo/query/acc.cgi?acc=GSE28352
- **RUDLOFF, S. ; KEMLER, R.** Differential requirements for beta-catenin during mouse development. *Development,* 2012, vol. 139, 3711-3721
- **TERRYN, S. et al.** A primary culture of mouse proximal tubular cells, established on collagen-coated membranes. *Am J Physiol Renal Physiol,* 2007, vol. 293, F476-485
- **REGOECZI et al.** *Am J Physiol.,* 1989, vol. 256 (1), E447-52